# EUROPEAN PATENT APPLICATION

(11) **EP 2 286 808 A1**
(43) Date of publication of application: **23.02.2011**
(21) Application number: 09168107.2
(22) Date of filing: 18.08.2009
(51) Int. Cl.: A61K 31/357, A61K 31/36, A61K 31/4192, A61K 31/4196, A61K 31/423, A61K 31/4245, A61K 31/433, A61K 31/4365, A61K 31/4709, A61K 31/517, A61K 31/519, A61K 31/5415, A61K 31/55, C07D 249/12, C07D 261/20, C07D 317/68, C07D 319/18

(54) **Cytohesin inhibitors**

(71) Applicant: Rheinische Friedrich-Wilhelms Universität, 53113 Bonn (DE)
(72) Inventor: Bajorath, Jürgen, 53113 Bonn (DE); Famulok, Michael, 53175 Bonn (DE); Kolanus, Waldemar, 53127 Bonn (DE); Hoch, Michael, 53913 Buschhoven (DE); Stumpfe, Dagmar, 53115 Bonn (DE); Bill, Anke, 53121 Bonn (DE); Novak, Nina Maria, 53119 Bonn (DE); Loch, Gerrit, 53115 Bonn (DE)
(74) Representative: Althausen, Sonja

(57) **Abstract**

The present invention relates to a pharmaceutical preparation comprising as an active ingredient a primary amide according to the general formula (1) and/or a secondary amide according to the general formula (2) and their use as a medicament.

## Description

The present invention relates to a pharmaceutical preparation comprising as an active ingredient a primary amide and/or a secondary amide. In particular, the present invention relates to the use of derivatives of primary amides and/or secondary amides for the treatment of autoimmune diseases, tumor diseases and/or for immunosuppression.

Cytohesins are small guanine nucleotide exchange factors (GEFs) that stimulate ADP ribosylation factors, ubiquitously expressed Ras-like GTPases, which control various cellular regulatory networks. Members of the cytohesin family include cytohesin-1, cytohesin-2 (ARNO), cytohesin-3, also known as Grp-1 in humans and Steppke in Drosophila, and cytohesin-4. All four currently known cytohesins share a conserved multi-domain structure, i.e. a Sec7-domain harbouring the GEF activity, a pleckstrin-homology domain, and a coiled-coil domain.

Although small molecular probes that effectively interfere with different cytohesin functions are highly desirable, only very few effective inhibitory chemotypes have thus far been described, as described in German patent application DE 10 2004 055 998. However, these inhibitors show only minor effectiveness, the most effective inhibitory chemotype being a pan-active 1-, 2-, 4-substituted triazole, which targets the Sec7 domain of cytohesins-1, -2, and -3, and inhibits their GEF activity and cytohesin-associated functions including insulin signalling. Further, it would be desirable to have inhibitors showing selectivity in the interference with cytohesin functions.

Therefore, the objective underlying the present invention was to provide novel compounds being usable as inhibitors of the GEFs of the cytohesin family in pharmacological treatments. UD 40170 / SAM:AL

The problem is solved by a pharmaceutical preparation comprising as an active ingredient a primary amide according to the general formula (1) and/or a secondary amide according to the general formula (2) as indicated below and/or racemates, enantiomers, diastereomers, solvates, hydrates, and pharmaceutically acceptable salts and/or esters thereof: 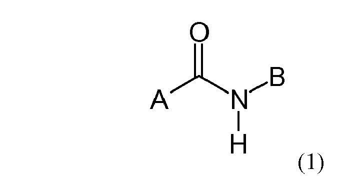 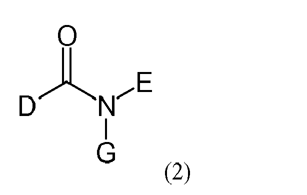 wherein:
- A: is selected from the group comprising linear or branched C₁-C₁₀-alkyl, linear or branched C₂-C₁₀-alkenyl and/or structural elements (A1), (A2), (A3), (A4), (A5), (A6), (A7), (A8), (A9) and/or (A10) as given as follows: 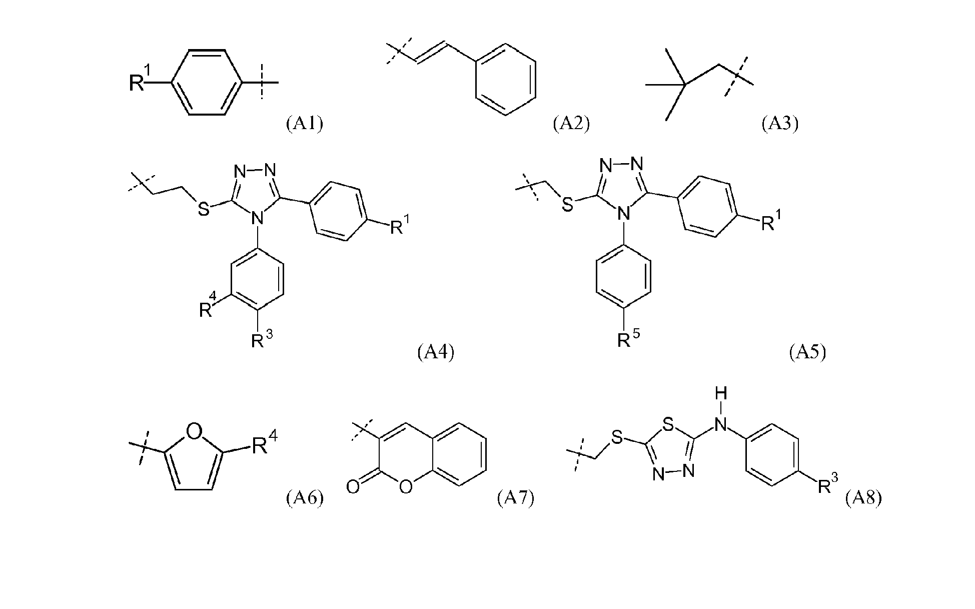 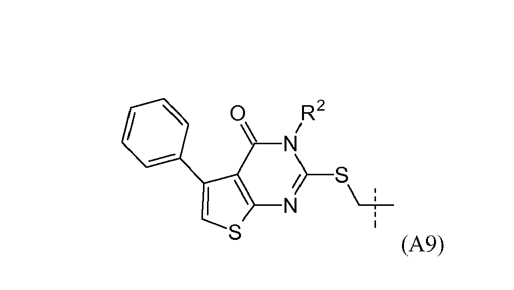 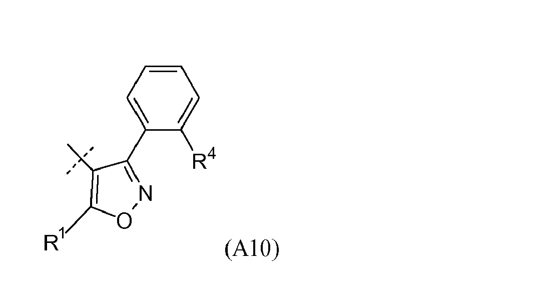
- B: is selected from the group comprising structural elements (B1), (B2), (B3), (B4), (B5), (B6), (B7), (B8), (B9), (B10), (B11), (B12) and/or (B13) as given as follows: 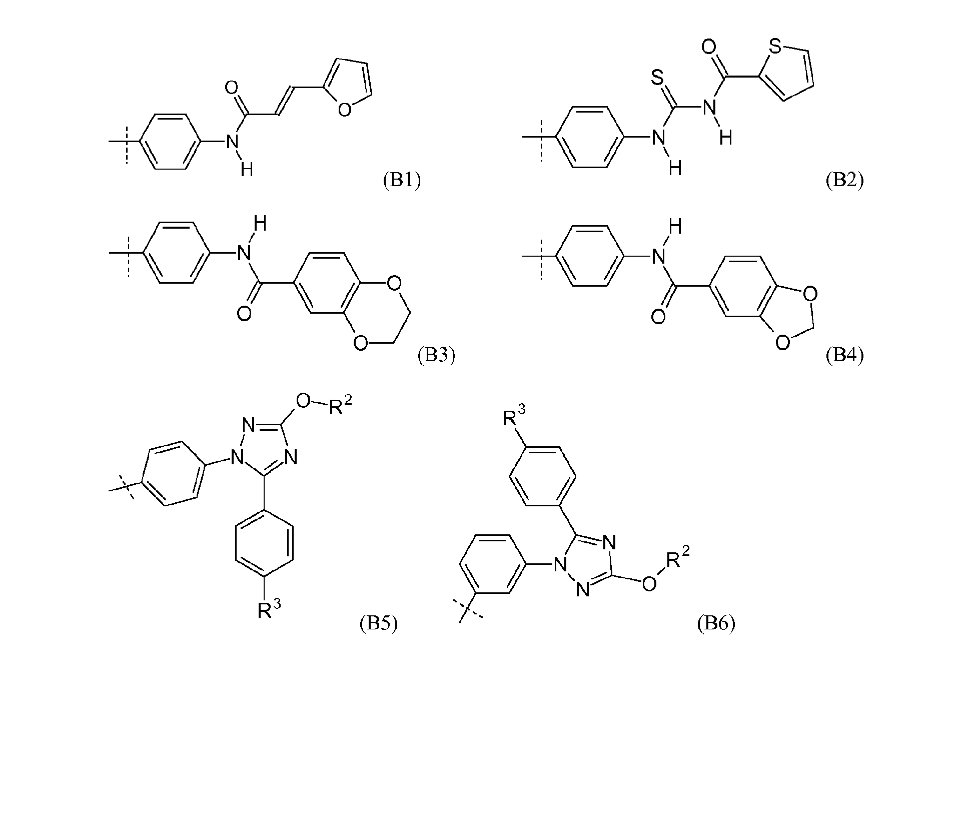 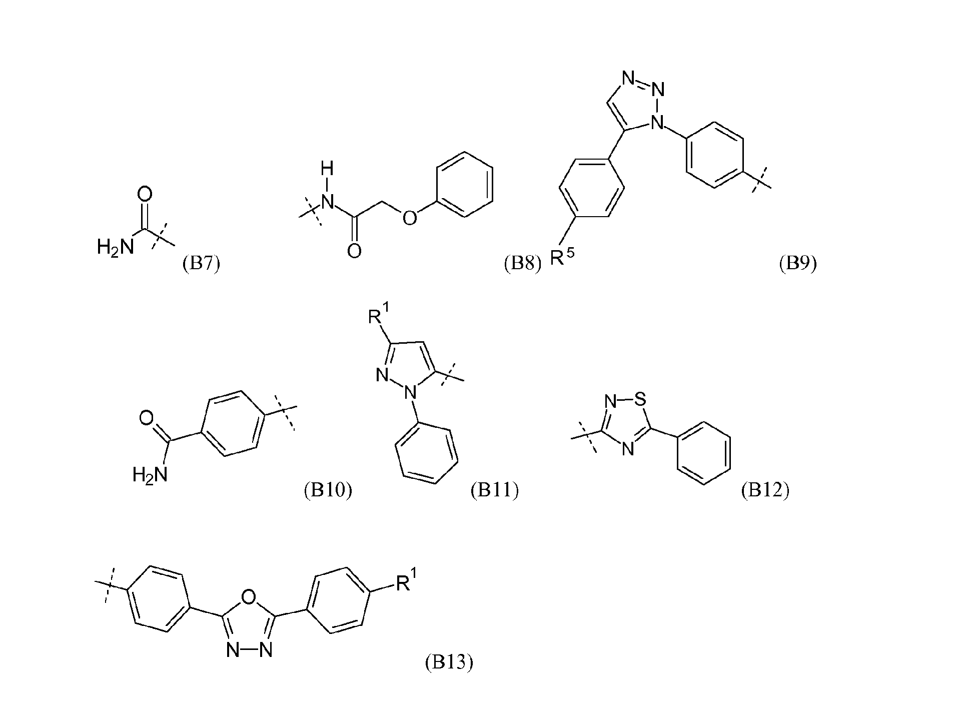
- D: is selected from the group comprising structural elements (D1), (D2), (D3), (D4) and/or (D5) as given as follows: 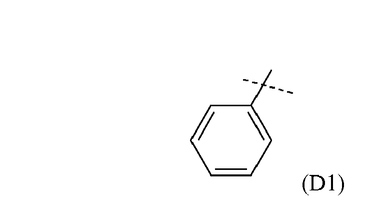 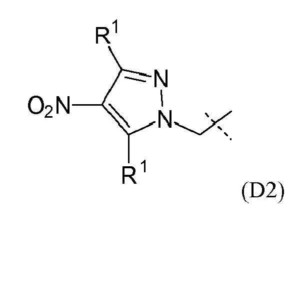 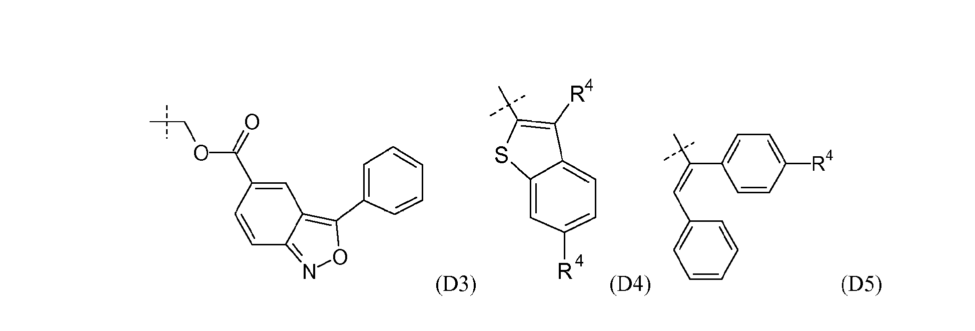
- E: is selected from the group comprising linear or branched C₁-C₁₀-alkyl, linear or branched C₂-C₁₀-alkenyl and/or structural elements (E1) or (E2) as given as follows: 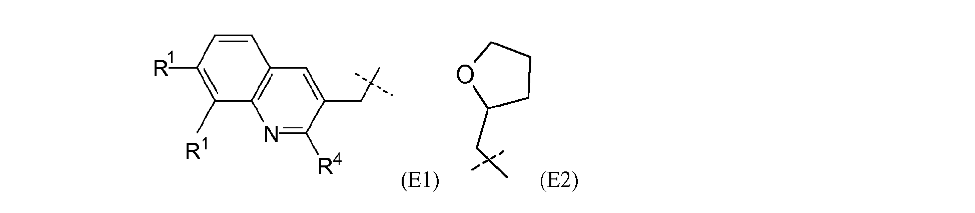
- G: is selected from the group comprising linear or branched C₁-C₁₀-alkyl, linear or branched C₂-C₁₀-alkenyl and/or structural elements (G1), (G2) or (G3) as given as follows: 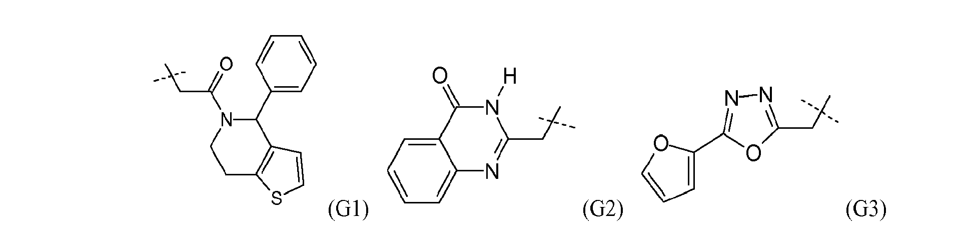 or
- E and G: together with the nitrogen atom to which they are attached form a 5- to 7- membered aromatic or non aromatic heterocycle selected from the group comprising structural elements (EG1) or (EG2) as given as follows: 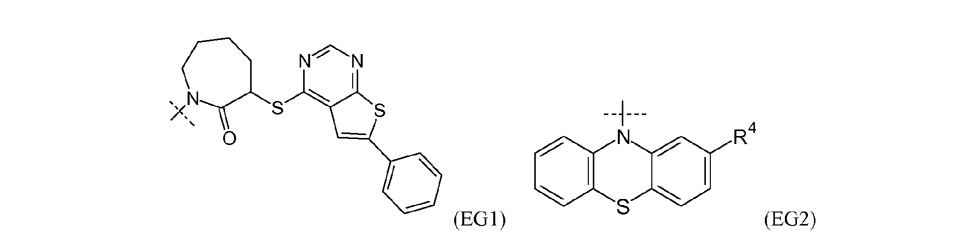 or
- D and G: together with the nitrogen atom to which they are attached form a 5- to 7- membered aromatic or non aromatic heterocycle (DG1) as given as follows: 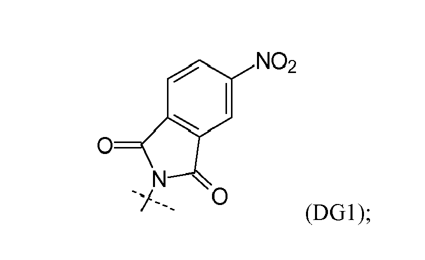
- R¹: is selected, the same or each independently of the others, from the group comprising hydrogen, linear or branched C₁-C₁₀-alkyl and/or linear or branched C₂-C₁₀-alkenyl;
- R²: is selected, the same or each independently of the others, from the group comprising linear or branched C₁-C₁₀-alkyl and/or linear or branched C₂-C₁₀-alkenyl;
- R³: is selected, the same or each independently of the others, from the group comprising linear or branched C₁-C₁₀-alkyl and/or linear or branched C₁-C₁₀-alkoxy;
- R⁴: is selected, the same or each independently of the others, from the group comprising hydrogen and/or halogen, preferably selected from the group comprising Cl, Br, F;
- R⁵: is selected, the same or each independently of the others, from the group comprising hydrogen and/or linear or branched C₁-C₁₀-alkoxy.

It was surprisingly found that the primary amides according to the general formula (1) and the secondary amides according to the general formula (2) exhibit a stronger inhibition of the guanine nucleotide exchange factors of the family of cytohesins, show a higher ability to interfere with cytohesin-dependent insulin signalling and/or exhibit a better effectiveness to block cytohesin-mediated cell adhesion of human leukocytes compared to the pan-active 1-, 2-, 4-substituted triazole known in the state of the art.

The term "alkyl" according to the invention is to be understood as meaning straight-chain or branched alkyl groups. The term "C₁-C₁₀-alkyl" as used herein refers to straight-chain or branched alkyl groups having 1 to 10 carbon atoms. Preferred C₁-C₁₀-alkyl groups are selected from the group comprising methyl, ethyl and the isomers of propyl, butyl, pentyl, hexyl, heptyl or octyl, such as, for example, isopropyl, isobutyl, tert.-butyl, sec.-butyl and/or isopentyl. Especially preferred are C₁-C₅-alkyl groups selected from the group comprising methyl, ethyl, isopropyl and/or tert.-butyl.

The term "alkenyl" according to the invention is to be understood as meaning straight-chain or branched alkyl groups having at least one or several double bonds. The term "C₂-C₁₀-alkenyl" as used herein refers to straight-chain or branched alkenyl groups having 2 to 10 carbon atoms and at least one or several double bonds. A preferred C₂-C₁₀-alkenyl is propen-1-yl.

The term "alkoxy" according to the invention is to be understood as meaning an alkyl group connected to the oxy connecting atom unless specifically stated otherwise. The term "C₁-C₁₀-alkoxy" as used herein refers to an alkyloxy group having 1 to 10 carbon atoms. C₁-C₁₀-alkyloxy group are preferably selected from the group comprising methoxy, ethoxy, n-propoxy, isopropoxy, n-butoxy, iso-butoxy, secondary-butoxy and/or tertiary-butoxy. Especially preferred C₁-C₁₀-alkoxy groups are selected from the group comprising methoxy, ethoxy and/or isopropoxy.

The term "halogen" according to the invention is to be understood as meaning fluorine, chlorine, bromine or iodine, preferably fluorine, chlorine or bromine.

In preferred embodiments, the pharmaceutical preparation comprises primary amides according to the general formula (1), wherein the structural element A is (A1) and the structural element B is selected from the group comprising (B1), (B2), (B3) and/or (B4).

Advantageously, the primary amides according to the general formula (1), wherein the structural element A is (A1) and the structural element B is selected from the group comprising (B1), (B2), (B3) and/or (B4) showed to be potent inhibitors in nucleotide exchange assays and blocking of cytohesin-mediated cell adhesion of human leukocytes.

In more preferred embodiments, the pharmaceutical preparation comprises primary amides selected from the group comprising the general formulas (3) and/or (4) as indicated below and/or racemates, enantiomers, diastereomers, solvates, hydrates, and pharmaceutically acceptable salts and/or esters thereof: 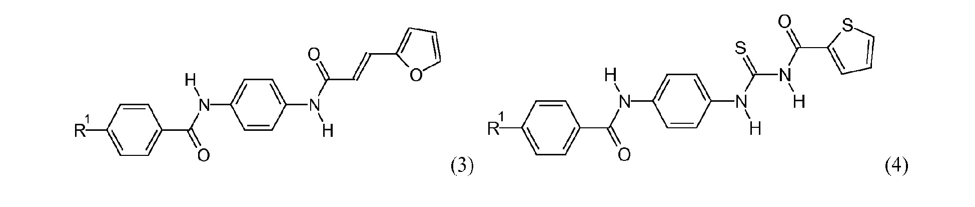 wherein:
- R¹: is selected, the same or each independently of the others, from the group comprising hydrogen, linear or branched C₁-C₁₀-alkyl and/or linear or branched C₂-C₁₀-alkenyl.

Preferably, the substituent R¹ of the primary amides according to the general formulas (3) and/or (4) is hydrogen or a C₁-C₅-alkyl group selected from the group comprising methyl, ethyl, isopropyl and/or tert.-butyl. Most preferably, substituent R¹ of the primary amides according to the general formulas (3) and/or (4) is hydrogen or tert.-butyl.

Advantageously, the primary amides according to the general formulas (3) and (4) can exhibit a good inhibition of the guanine nucleotide exchange factors of the family of cytohesins, a high ability to interfere with cytohesin-dependent insulin signalling, and a good effectiveness to block cytohesin-mediated cell adhesion of human leukocytes.

It was surprisingly found that the primary amides according to the general formulas (3) and (4) can have a significantly improved effect if the substituent R¹ is a smaller alkyl group or hydrogen. Thus it was surprisingly determined that a significant increase in efficacy in the inhibitory effects of the primary amide according to the general formula (3) can be achieved when the substituent R¹ is hydrogen.

In very especially preferred embodiments, the pharmaceutical preparation comprises primary amides selected from the group comprising the general formulas (5) and/or (6) as indicated below and/or racemates, enantiomers, diastereomers, solvates, hydrates, and pharmaceutically acceptable salts and/or esters thereof:

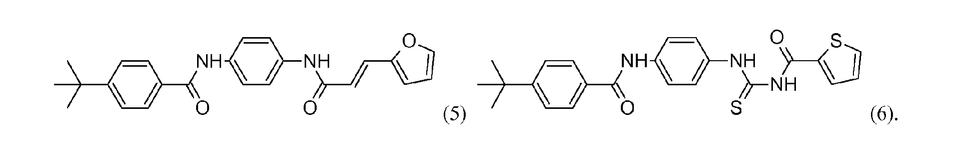

Advantageously, the primary amides according to the general formulas (5) and (6) can exhibit a stronger inhibition of the guanine nucleotide exchange factors of the family of cytohesins, a higher ability to interfere with cytohesin-dependent insulin signalling, and a better effectiveness to block cytohesin-mediated cell adhesion of human leukocytes compared to the pan-active 1-, 2-, 4-substituted triazole known in the state of the art.

In other preferred embodiments, the pharmaceutical preparation comprises primary amides according to the general formula (1), wherein the structural element A is selected from the group comprising branched C₁-C₁₀-alkyl groups, (A2) and/or (A3), preferably branched C₁-C₁₀-alkyl groups or (A3), and the structural element B is selected from the group comprising (B5) and/or (B6).

Advantageously, the primary amides according to the general formula (1), wherein the structural element A is selected from the group comprising branched C₁-C₁₀-alkyl groups and/or (A3), and the structural element B is selected from the group comprising (B5) and/or (B6) can exhibit a selective activity in down-regulating insulin signaling.

In other preferred embodiments, the pharmaceutical preparation comprises primary amides according to the general formula (1), wherein the structural element A is (A4), and the structural element B is (B7).

Advantageously, the primary amides according to the general formula (1), wherein the structural element A is (A4), and the structural element B is (B7) can exhibit a selective activity in inhibiting cytohesin-mediated cell adhesion of human leukocytes and/or inhibition of the guanine nucleotide exchange factors of the family of cytohesins.

In other preferred embodiments, the pharmaceutical preparation comprises primary amides according to the general formula (1), wherein the structural element A is (A5), and the structural element B is selected from the group comprising (B8) and/or (B10).

In other preferred embodiments, the pharmaceutical preparation comprises primary amides according to the general formula (1), wherein the structural element A is selected from the group comprising (A6), (A7) and/or (A10), and the structural element B is (B9).

In other preferred embodiments, the pharmaceutical preparation comprises primary and/or secondary amides selected from the group comprising compounds (7) to (30) as indicated below and/or racemates, enantiomers, diastereomers, solvates, hydrates, and pharmaceutically acceptable salts and/or esters thereof:

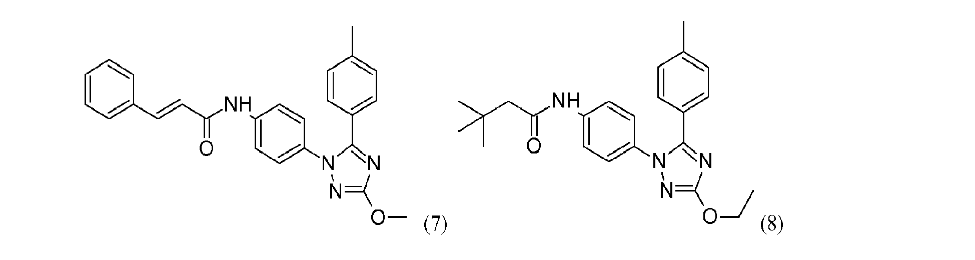

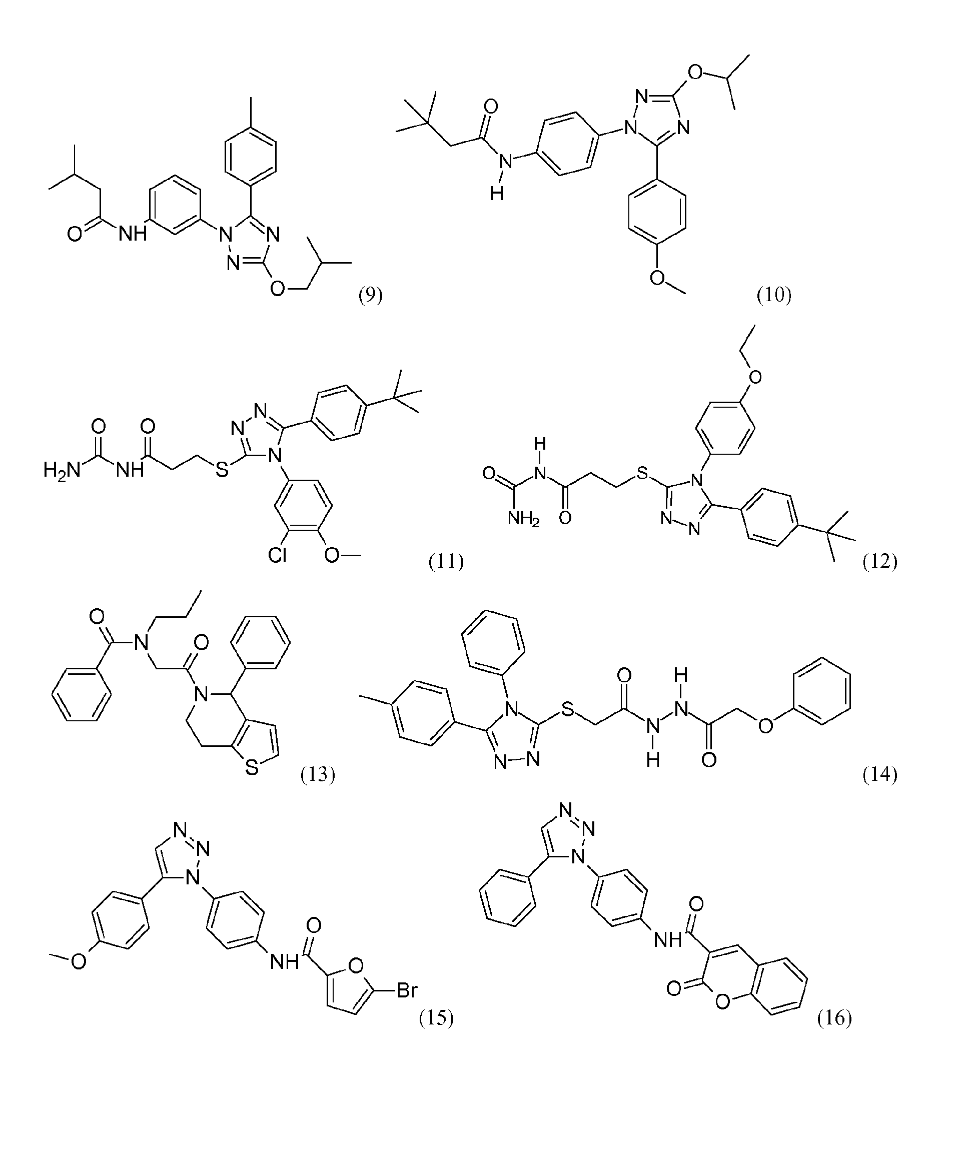

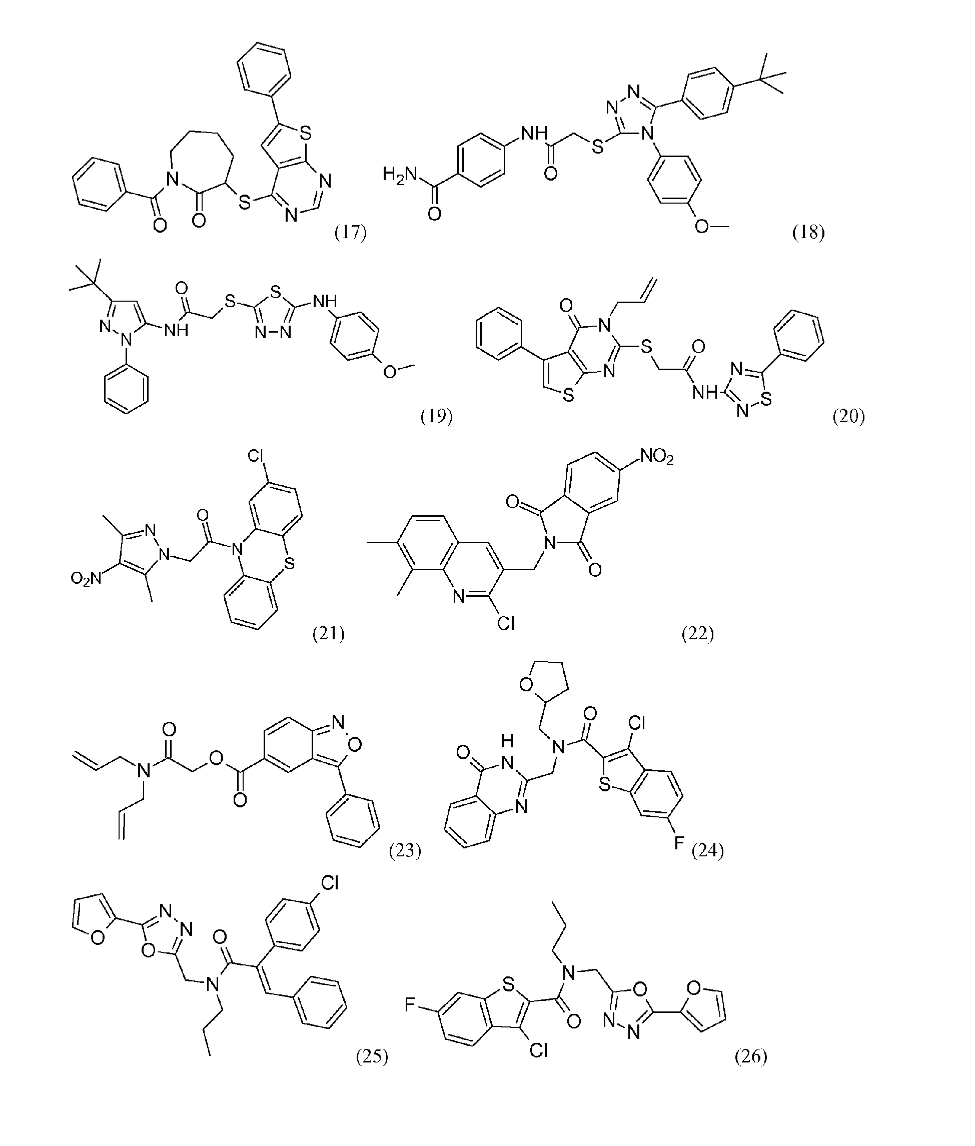

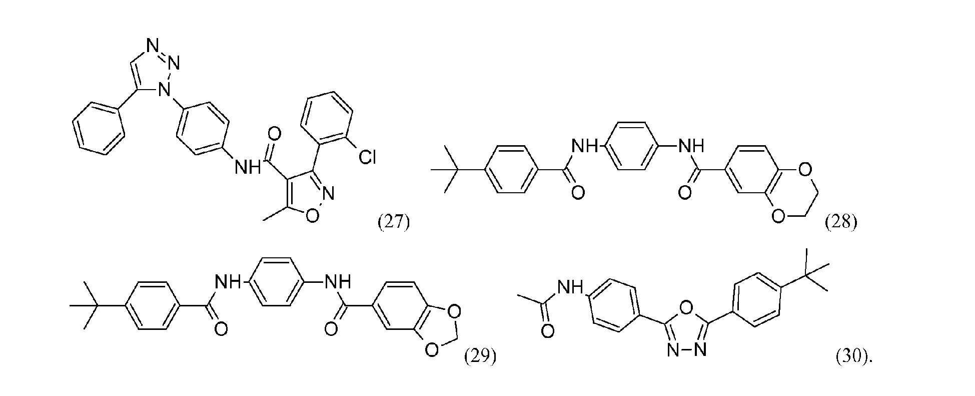

Advantageously, the primary and secondary amides according to the general formulas (7) to (30) can exhibit a selective activity. The primary amides according to the general formulas (20), (5), and (6) showed to be potent inhibitors in nucleotide exchange essays, wherein the primary amide according to the general formula (20) showed to have a dual activity in cell adhesion and nucleotide exchange assays. The primary amides according to the general formulas (11) and (29) exhibited a dual activity in cell adhesion and nucleotide exchange assays.

Very advantageously, the primary amides according to the general formulas (8), (9), and (10) were selectively active in down-regulating insulin signalling, the primary amides according to the general formula (12) showed a selective inhibition of cell adhesion.

Further advantageously, the amides according to the general formulas (25), (12), (26), and (30) were identified being strong cell adhesion inhibitors, wherein in particular the amides according to the general formulas (25), and (30), were found to be strong inhibitors of cytohesin-mediated cell adhesion. This allows thus for further extending the potential to analyze cytohesin functions using small molecules.

A further aspect of the present invention relates to primary amides according to the general formula (1) and/or a secondary amides according to the general formula (2) and/or racemates, enantiomers, diastereomers, solvates, hydrates, pharmaceutically acceptable salts and/or esters thereof for use as a medicament.

Preferably, primary amides according to the general formula (1), wherein the structural element A is selected from the group comprising branched C₁-C₁₀-alkyl groups, (A2) and/or (A3), preferably branched C₁-C₁₀-alkyl groups or (A3), and the structural element B is selected from the group comprising (B5) and/or (B6), and/or primary amides according to the general formula (1), wherein the structural element A is (A4), and the structural element B is (B7), and/or primary amides according to the general formula (1), wherein the structural element A is (A5), and the structural element B is selected from the group comprising (B8) and/or (B10), and/or primary amides according to the general formula (1), wherein the structural element A is selected from the group comprising (A6), (A7) and/or (A10), and the structural element B is (B9) and/or racemates, enantiomers, diastereomers, solvates, hydrates, and pharmaceutically acceptable salts and/or esters thereof are usable as a medicament.

Preferably, amides selected from the group comprising primary amides according to the general formulas (3) and/or (4), especially formulas (5) and/or (6), primary and/or secondary amides selected from the group comprising amides according to the formulas (7) to (30) and/or racemates, enantiomers, diastereomers, solvates, hydrates, pharmaceutically acceptable salts and/or esters thereof are usable as a medicament.

Another aspect of the present invention relates to the primary amides according to the general formula (1) and/or a secondary amides according to the general formula (2) and/or racemates, enantiomers, diastereomers, solvates, hydrates, and pharmaceutically acceptable salts and/or esters thereof for use in the therapeutic and/or prophylactic treatment of a disease selected from the group comprising autoimmune diseases such as rheumatoid arthritis, multiple sclerosis, psoriasis, Crohn's disease, allergies, mastocytosis, tumor diseases such as lung or bronchial cancer, colon, gastrointestinal, pancreatic or rectal cancer, prostate cancer, lymphatic cancer or leukemia, bladder or kidney cancer, breast and/or ovarian cancer, diseases and pathological conditions that are linked to a hyperfunction of cytohesin dependent receptor tyrosine kinases such as the ErbB receptors and other growth factor receptors selected from the group comprising developmental syndromes such as craniosynostosis, dwarfism, Hirschsprung disease, multiple endocrine neoplasia, vascular dysmorphogenesis and/or disorders of the lymphatic system, diseases and pathological conditions that are linked to a regulation of the insulin and/or insulin-like growth factor (IGF) signalling pathway selected from the group comprising obesity, cell aging, age-related cell damage, especially in the liver and/or the pancreas, age-related pathological conditions of liver and/or pancreatic cells, age-related functional disorders in the liver and/or pancreas, cell stress, especially oxidative stress, especially stress induced as a result of increased sugar metabolization, and/or apoptosis, especially β-celt apoptosis, for immunosuppression, for example, in cases of organ transplants, and/or for inhibition of angiogenesis.

Preferably, primary amides according to the general formula (1), wherein the structural element A is selected from the group comprising branched C₁-C₁₀-alkyl groups, (A2) and/or (A3), preferably branched C₁-C₁₀-alkyl groups or (A3), and the structural element B is selected from the group comprising (B5) and/or (B6), and/or primary amides according to the general formula (1), wherein the structural element A is (A4), and the structural element B is (B7), and/or primary amides according to the general formula (1), wherein the structural element A is (A5), and the structural element B is selected from the group comprising (B8) and/or (B10), and/or primary amides according to the general formula (1), wherein the structural element A is selected from the group comprising (A6), (A7) and/or (A10), and the structural element B is (B9) and/or racemates, enantiomers, diastereomers, solvates, hydrates, and pharmaceutically acceptable salts and/or esters thereof are usable for the therapeutic and/or prophylactic treatment of the aforementioned diseases.

In preferred embodiments, amides selected from the group comprising primary amides according to the general formulas (3) and/or (4), especially formulas (5) and/or (6), primary and/or secondary amides selected from the group comprising amides according to the formulas (7) to (30) and/or racemates, enantiomers, diastereomers, solvates, hydrates, and pharmaceutically acceptable salts and/or esters thereof are usable for the therapeutic and/or prophylactic treatment of the aforementioned diseases.

Preferred diseases are selected from the group comprising autoimmune diseases such as rheumatoid arthritis, multiple sclerosis, psoriasis, Crohn's disease, allergies, mastocytosis, tumor diseases such as lung or bronchial cancer, colon, gastrointestinal, pancreatic or rectal cancer, prostate cancer, lymphatic cancer or leukemia, bladder or kidney cancer, breast and/or ovarian cancer, diseases and pathological conditions that are linked to a hyperfunction of cytohesin dependent receptor tyrosine kinases such as the ErbB receptors and other growth factor receptors selected from the group comprising developmental syndromes such as craniosynostosis, dwarfism, Hirschsprung disease, multiple endocrine neoplasia, vascular dysmorphogenesis or disorders of the lymphatic system and/or obesity.

In also very preferred embodiments, the primary amides according to the general formula (1) and/or a secondary amides according to the general formula (2) are usable for immunosuppression, for example, in cases of organ transplants.

The present invention also relates to the use of primary amides according to the general formula (1) and/or secondary amides according to the general formula (2) and/or racemates, enantiomers, diastereomers, solvates, hydrates, and pharmaceutically acceptable salts and/or esters thereof for the manufacture of a medicament.

Preferably, primary amides according to the general formula (1), wherein the structural element A is selected from the group comprising branched C₁-C₁₀-alkyl groups, (A2) and/or (A3), preferably branched C₁-C₁₀-alkyl groups or (A3), and the structural element B is selected from the group comprising (B5) and/or (B6), and/or primary amides according to the general formula (1), wherein the structural element A is (A4), and the structural element B is (B7), and/or primary amides according to the general formula (1), wherein the structural element A is (A5), and the structural element B is selected from the group comprising (B8) and/or (B10), and/or primary amides according to the general formula (1), wherein the structural element A is selected from the group comprising (A6), (A7) and/or (A10), and the structural element B is (B9) and/or racemates, enantiomers, diastereomers, solvates, hydrates, and pharmaceutically acceptable salts and/or esters thereof are usable for the manufacture of a medicament.

In preferred embodiments, the present invention relates to the use of amides selected from the group comprising primary amides according to the general formulas (3) and/or (4), especially formulas (5) and/or (6), primary and/or secondary amides selected from the group comprising amides according to the formulas (7) to (30) and/or racemates, enantiomers, diastereomers, solvates, hydrates, and pharmaceutically acceptable salts and/or esters thereof for the manufacture of a medicament.

A further aspect of the present invention relates to the use of primary amides according to the general formula (1) and/or a secondary amides according to the general formula (2) and/or racemates, enantiomers, diastereomers, solvates, hydrates, and pharmaceutically acceptable salts and/or esters thereof for the manufacture of a pharmaceutical preparation for the therapeutic and/or preventive treatment of a disease selected from the group comprising autoimmune diseases such as rheumatoid arthritis, multiple sclerosis, psoriasis, Crohn's disease, allergies, mastocytosis, tumor diseases such as lung or bronchial cancer, colon, gastrointestinal, pancreatic or rectal cancer, prostate cancer, lymphatic cancer or leukemia, bladder or kidney cancer, breast and/or ovarian cancer, diseases and pathological conditions that are linked to a hyperfunction of cytohesin dependent receptor tyrosine kinases such as the ErbB receptors and other growth factor receptors selected from the group comprising developmental syndromes such as craniosynostosis, dwarfism, Hirschsprung disease, multiple endocrine neoplasia, vascular dysmorphogenesis and/or disorders of the lymphatic system, diseases and pathological conditions that are linked to a regulation of the insulin and/or insulin-like growth factor (IGF) signalling pathway selected from the group comprising obesity, cell aging, age-related cell damage, especially in the liver and/or the pancreas, age-related pathological conditions of liver and/or pancreatic cells, age-related functional disorders in the liver and/or pancreas, cell stress, especially oxidative stress, especially stress induced as a result of increased sugar metabolization, and/or apoptosis, especially β-celt apoptosis for immunosuppression, for example, in cases of organ transplants, and/or for inhibition of angiogenesis.

The term "prophylactic treatment" of a disease according to the invention is to be understood as meaning that the compositions according to the invention can be applied before symptoms of the disease are manifest. Especially, the term "prophylactic treatment" of a disease is to be understood as meaning a medical treatment.

One particular advantage of the amides that can be used according to the invention is that the amides can especially also be used in preventive applications. This makes it possible to use the amides not only to treat existing pathological conditions, but also for preventive applications, for example to prevent age-related cell damage or obesity. Of particular advantage is the fact that, for example, obesity can be avoided through preventive treatment.

Advantageously, the primary and/or secondary amides according to the general formulas (1) and (2) can be especially useful for the treatment of diseases for which an inhibitor of the GEFs of the cytohesin family is indicated, and especially diseases and pathological conditions that are linked to a hyperfunction of cytohesin dependent receptor tyrosine kinases such as the ErbB receptors and other growth factor receptors selected from the group comprising tumor diseases such as lung or bronchial cancer, colon, gastrointestinal, pancreatic or rectal cancer, prostate cancer, lymphatic cancer or leukemia, bladder or kidney cancer, breast and/or ovarian cancer, and/or developmental syndromes such as craniosynostosis, dwarfism, Hirschsprung disease, multiple endocrine neoplasia, vascular dysmorphogenesis and/or disorders of the lymphatic system.

Preferably, primary amides according to the general formula (1), wherein the structural element A is selected from the group comprising branched C₁-C₁₀-alkyl groups, (A2) and/or (A3), preferably branched C₁-C₁₀-alkyl groups or (A3), and the structural element B is selected from the group comprising (B5) and/or (B6), and/or primary amides according to the general formula (1), wherein the structural element A is (A4), and the structural element B is (B7), and/or primary amides according to the general formula (1), wherein the structural element A is (A5), and the structural element B is selected from the group comprising (B8) and/or (B10), and/or primary amides according to the general formula (1), wherein the structural element A is selected from the group comprising (A6), (A7) and/or (A10), and the structural element B is (B9) and/or racemates, enantiomers, diastereomers, solvates, hydrates, and pharmaceutically acceptable salts and/or esters thereof are usable for the manufacture of a pharmaceutical preparation for the therapeutic and/or prophylactic treatment of the aforementioned diseases.

In preferred embodiments, amides selected from the group comprising primary amides according to the general formulas (3) and/or (4), especially formulas (5) and/or (6), primary and/or secondary amides selected from the group comprising amides according to the formulas (7) to (30) and/or racemates, enantiomers, diastereomers, solvates, hydrates, and pharmaceutically acceptable salts and/or esters thereof are usable for the manufacture of a pharmaceutical preparation for the therapeutic and/or prophylactic treatment of the aforementioned diseases.

In preferred embodiments the disease is selected from the group comprising autoimmune diseases such as rheumatoid arthritis, multiple sclerosis, psoriasis, Crohn's disease, allergies, mastocytosis, tumor diseases such as lung or bronchial cancer, colon, gastrointestinal, pancreatic or rectal cancer, prostate cancer, lymphatic cancer or leukemia, bladder or kidney cancer, breast and/or ovarian cancer and/or diseases and pathological conditions that are linked to a hyperfunction of cytohesin dependent receptor tyrosine kinases such as the ErbB receptors and other growth factor receptors selected from the group comprising developmental syndromes such as craniosynostosis, dwarfism, Hirschsprung disease, multiple endocrine neoplasia, vascular dysmorphogenesis and/or disorders of the lymphatic system.

Further advantageously, the primary and/or secondary amides according to the general formulas (1) and (2) can be useful for the therapeutic and/or preventive treatment of diseases and pathological conditions that are linked to a regulation of the insulin and/or insulin-like growth factor (IGF) signaling pathway. In preferred embodiments the diseases or pathological condition that is linked to a regulation of the insulin and/or insulin-like growth factor (IGF) signaling pathway is obesity.

In also very preferred embodiments, the primary and/or a secondary amides according to the general formulas (1) and (2) are usable for immunosuppression, for example, in cases of organ transplants or for inhibition of angiogenesis.

A further aspect of the present invention relates to the use of primary amides according to the general formula (1) and/or a secondary amides according to the general formula (2) and/or racemates, enantiomers, diastereomers, solvates, hydrates, and pharmaceutically acceptable salts and/or esters thereof for chemically inducing longevity or increasing the lifespan of mammals. Especially primary amides according to the general formulas (3) and/or (4), especially formulas (5) and/or (6), primary and/or secondary amides selected from the group comprising amides according to the formulas (7) to (30) and/or racemates, enantiomers, diastereomers, solvates, hydrates, and pharmaceutically acceptable salts and/or esters thereof are usable for chemically inducing longevity or increasing the lifespan of mammals.

The term "chemically induced longevity" within the context of this invention means that by administering the amides according to the invention, the lifespan of an organism and/or a tissue or organ can be extended. An extension of the lifespan of an organism and/or of a tissue or organ can advantageously be achieved by administering an amide without necessitating surgical treatment or a genetic alteration of the organism; in other words, longevity can be induced chemically by administering a substance.

Advantageously, the amides can have only a slight or negligible toxicity when administered. This enables their long-term use, for example. It also enables their administration for preventive purposes, especially in humans.

The pharmaceutical preparation can thus be administered with usual methods such as, for example, orally, dermally and/or intravenously. Oral or parenteral administration is preferred, with oral administration being especially preferred. In preferred embodiments, the pharmaceutical preparation is formulated for oral or intravenous administration.

The Examples which follow serve to illustrate the invention in more detail but do not constitute a limitation thereof.

The figures show:
Figure 1 the relative exchange rate from guanidine-diphosphate (GDP) to guanidine-triphosphate (GTP) in the presence of the amides according to formulas (5) to (30) in comparison to the exchange rate in the presence of the control SecinH3 (K);
Figure 2 Regulation of d4E-BP mRNA levels due to interfering with insulin signaling in *Drosophila* S2 cells with amides according to formulas (5) to (30) in comparison to the control SecinH3 (K);
Figure 3 the Inhibition of cell adhesion of Jurkat E6 cells to an immobilized ICAM-1-Fc-fusion protein after stimulation with the monoclonal antibody OKT3 and phorbolester (PMA) of the amides according to formulas (5) to (30) in comparison to the control SecinH3 (K).

### Example 1

The inhibition of the exchange activity of the sec7-domains of cytohesin-2 (ARNO) of the amides according to formulas (5) to (30) was determined by a guanine nucleotide exchange assay.

For this purpose, the sec7-domains of cytohesin-2 (ARNO-Sec7) and [Δ17]ARF1, a mutant of the ADP-ribosylation factor (ARF) protein, were subcloned into pET15 vectors (Novagen) as described in Hafner, M. et al. Nature 444, 941-944 (2006). N-terminal truncated [Δ17]ARF1 (amino acids 18-181), lacking the first 17 amino acids and ARNO-Sec7 (amino acids 50-255 of ARNO) were expressed in *Escherichia coli* and purified by Ni-NTA chromatography (Ni-NTA agarose, Qiagen).

The exchange from guanidine-diphosphate (GDP) to guanidine-triphosphate (GTP) induces a conformation change of the ribosylation factor, as a result of which the fluorescence of a tryptophan radical of the protein is increased. GDP/GTP exchange was measured on [Δ17]ART1 by tryptophan fluorescence because a large increase in intrinsic fluorescence of ARF occurs upon exchange of GDP for GTP. All measurements were performed in PBS (137 mM NaCl, 2.7 mM KCl, 10.2 mM Na₂HPO₄, 1.8 mM KH₂PO₄) pH 7.4, 3 mM MgCl₂ at 37°C.

[Δ17]ARF1 (1 µM) in PBS without MgCl₂ was preincubated with 80 µM GDP in the presence of 2 mM ethylenediaminetetraacetic acid (EDTA) for 15 minutes. The bound GDP was stabilized by addition of MgCl₂ at a final concentration of 3 mM and incubation for 5 minutes. For each exchange reaction 250 nM [Δ17]ARF1 was mixed with 10 nM ARNO-Sec7 (total volume 200 µl) in the absence or presence of inhibitoring amides according to formulas (5) to (30). The reaction was started by injection of 50 µM GTP.

The tryptophan fluorescence was measured at excitation and emission wavelength of 280 nm and 340 nm respectively. All fluorescent measurements were performed with a Varioskan microplate reader (Thermo Scientific), in 96-well plates. For analysis all data were fitted by linear regression.

The pan-active cytohesin inhibitor "SecinH3", a 1-, 2-, 4-substituted triazole according to formula (9) as disclosed in DE 10 2004 055 998 served as the control (K). The relative exchange rate of the control SecinH3 (K) was set to be 1.

The relative exchange rate was determined by comparing the initial linear rise of the fluorescence signal (exchange rate) in the presence of 5µM of the amides according to formulas (5) to (30) with the exchange rate in the presence of 5µM control SecinH3 (K), whereas the latter was set to be 1.

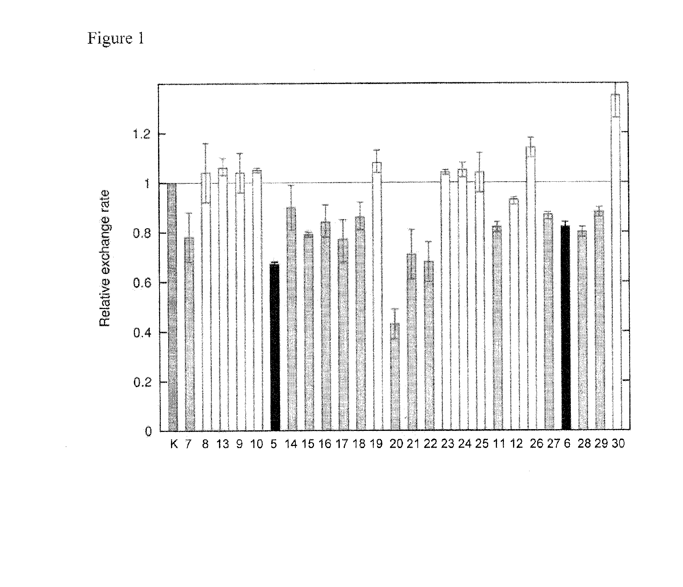

As can be seen in Fig. 1, several amides especially according to formulas (5), (6), (7), (11), (14) to (18), (20) to (22), and (27) to (29) exhibited an inhibition of the sec7-domain of the isolated cytohesin-2, which was better than that of the control SecinH3 (K).

Further, the IC₅₀ value inhibition of the sec7-domain of the isolated cytohesin-2 was determined. The IC₅₀ value was be determined by plotting the initial linear rise of the fluorescence signal against the added concentrations of the amide. The IC₅₀ value corresponds to the concentration of the amide at which the activity of the protein is reduced to half. The lower the IC₅₀ value, the more strongly the compound inhibits the sec7-domain.

The IC₅₀ value of the amides according to formulas (20), (5), and (6) measured for the isolated Sec-7 domain of cytohesin-2 was 2.1 µM, 3.1 µM, and 8.0 µM, respectively, compared to 11.4 µM for the reference SecinH3 (K). With an IC₅₀ value of 2.1 µM, the amide according to formula (20) was the most active new inhibitor in nucleotide exchange assays. Both amides according to formulas (5) and (20) inhibition displayed a dose-response behaviour, indicating specificity of the interactions.

### Example 2

### Drosophila assays:

In order to evaluate potential interference of the amides according to formulas (5) to (30) with *Drosophila* insulin signaling, S2 (Schneider 2 insect cell line, Invitrogen) cells were grown under starvation conditions and subsequently treated with insulin. This triggers a strong activation of the insulin signaling cascade resulting in the nuclear exclusion of the forkhead-box transcription factor dFOXO, which in turn down-regulates the expression of the transcriptional repressor d4E-BP (eIF4E-binding protein). If compounds inhibit the GEF activity of Steppke, insulin signaling is blocked and dFOXO is translocated into the nucleus, leading to an up-regulation of d4E-BP transcription. This was measured by quantitative RT-PCR and compared to the effect of the control SecinH3 (K) in the same assay.

### Cell culture and compound treatment:

0.5 x 10⁶ S2 cells were grown in 24-well dishes in medium (Schneiders Drosophila Medium, PAN Biotech) without FCS (heat-inactivated fetal calf serum) representing starvation condition. Six hours after seeding cells were treated for 18 hours with 10 µM of the respective amide according to formulas (5) to (30) or 10 µM SecinH3 (K) as reference. 22 hours after seeding cells were treated with 5 µg/ml insulin (Sigma). Controls were performed without amide and/or without insulin treatment. The amides were dissolved in dimethyl sulfoxide (DMSO). Final DMSO concentration in the cell culture medium was 0.5 %.

### RNA Isolation, cDNA synthesis and Real-Time PCR:

NucleoSpin 8 RNA Kit (Macherey-Nagel) was used for RNA preparation according to manufacturer's instructions. Genomic DNA digestion and first strand cDNA synthesis was carried out with 250 ng total RNA using QuantiTect Reverse Transkription Kit (Qiagen). Quantitative PCR was performed with the iQ5 Real-Time PCR detection system (Bio-Rad) and iQ SYBR Green Super Mix (Bio-Rad). Three reactions were done in parallel for each template. Real Time PCR was analysed using Bio-Rad iQ5 Optical System Software and Microsoft Excel.

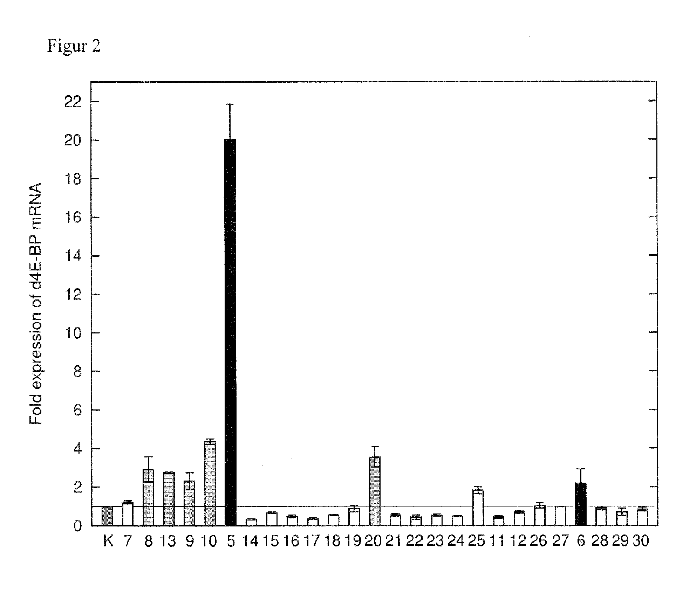

The d4E-BP mRNA level after treatment of cells with the amides according to formulas (5) to (30) compared to the control SecinH3 is shown in Fig. 2. The d4E-BP mRNA level after treatment of cells with the control SecinH3 (K) was set to be 1.

It can be seen from Fig. 2 that in *Drosophila* S2 cells treated with several amides, especially according to formulas (5), (6), (8), (9), (10), (13), and (20), d4E-BP transcript levels were increased. Especially the amide according to formula (5) induced a ∼20-fold increase in dFOXO-dependent d4E-BP transcript levels compared to the reference (K).

As the activity of the insulin signaling pathway can be determined from the transcription rate of the insulin target gene Drosophila eukaryotic initiation factor 4E binding protein (d4E-BP), these results show that the insulin signaling pathway is influenced by these amides in vitro.

### Example 3

### Cell adhesion assays

The ability of the amides according to formulas (5) to (30) to inhibit adhesion of the T cell line Jurkat E6 to an immobilized ICAM-1(Inter-Cellular Adhesion Molecule 1)-Fc-fusion protein was tested. On the T cell side, this interaction is exclusively mediated by the β-2 integrin LFA-1 that was enabled to bind to immobilized ICAM-1 through stimulation of Jurkat cells with an anti-TCR (T cell receptor) antibody or with the phorbol ester PMA (Phorbol 12-myristate 13-acetate). Cell adhesion to ICAM-Fc was carried out on 96-well dishes and was read out by fluorescence detection of total input versus bound cells which had been labeled with the DNA dye H33342.

Jurkat E6.1 cells were incubated in HBSS (Hank's BSS, PAA), 0.5% DMSO and, where indicated, 25 µM of the respective amide for one hour at 37°C, 5% CO₂. After 30 minutes the cells were labeled with the fluorochrome bisbenzimide trihydrochloride H33342 (Sigma-Aldrich) at 12 mg/ml for 30 minutes at 37°C. 2 x 10⁵ cells/ well were subsequently dispensed into a 96-well plate at 100µl/well. Prior to use plates were coated with 12µg/ml goat anti-human IgG for 90 minutes at 21°C, blocked with 1% BSA (Bovine serum albumin) in PBS overnight and incubated for 60 minutes with culture supernatants from CV-1 cells expressing an ICAM-1-Fc fusion protein.

Cells were either stimulated with 5 µg/ml purified anti CD3 antibody (OKT3, hybridome of ATCC, mAb OKT3 purified on protein A, according to standard methods from Current Protocols in Immunology, 5th Ed., Coligan et al., Edts, Wiley 2004) or with 50 ng/ml PMA, respectively. Cells were allowed to adhere to the ICAM-1-Fc coated dishes for 15 minutes and unbound cells were washed off with Hank's BSS (PAA). Adherent cells were read out using a fluorescence plate reader (Synergy-HT1, Biotek) at 485 nm in triplicates.

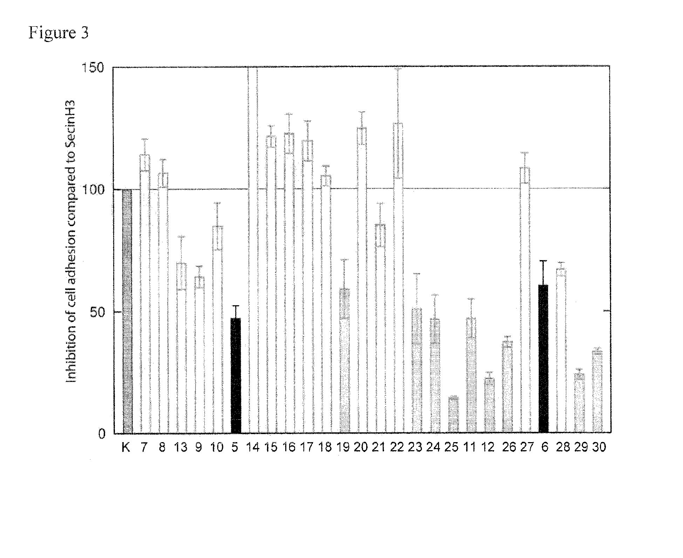

As can be seen in Fig. 3, several amides especially according to formulas (5), (6), (11), (12), (19), (23), (24), (25), (26), (29), and (30) inhibited the adhesion of the Jurkat E6.1 cells to ICAM-1 better than that of the control (K). "100% adhesion" corresponds to the mean of OKT3 and PMA stimulated samples treated with 25 µM control control Secin H3 (K) as reference.

These experiments show that especially the primary amides according to formulas (5) and (6) exhibit a stronger inhibition of the guanine nucleotide exchange factors of the family of cytohesins, a higher ability to interfere with cytohesin-dependent insulin signalling, and a better effectiveness to block cytohesin-mediated cell adhesion of human leukocytes compared to the control, the pan-active 1-, 2-, 4-substituted triazole known in the state of the art.

These experiments further show that the primary and secondary amides according to formulas (7) to (30) can exhibit a selective activity. Especially, the primary amides according to formulas (20), (5), and (6) showed to be potent inhibitors in nucleotide exchange essay, wherein the primary amide according to formula (20) showed to have a dual activity in cell adhesion and nucleotide exchange assays. The primary amides according to formulas (11) and (29) exhibited a dual activity in cell adhesion and nucleotide exchange assays.

Further, the primary amides according to the formulas (8), (9), and (10) were selectively active in down-regulating insulin signalling, and the primary amide according to formula (12) showed a selective inhibition of cell adhesion, while the amides according to formulas (25), (12), (26), and (30) were identified as being strong cell adhesion inhibitors, wherein in particular the amides according to the formulas (25), and (30), were found to be strong inhibitors of cytohesin-mediated cell adhesion.

## Claims

1. A pharmaceutical preparation comprising as an active ingredient a primary amide according to the general formula (1) and/or a secondary amide according to the general formula (2) as indicated below and/or racemates, enantiomers, diastereomers, solvates, hydrates, and pharmaceutically acceptable salts and/or esters thereof: 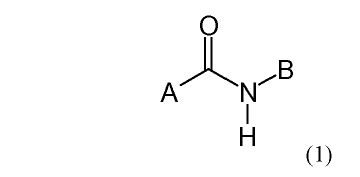 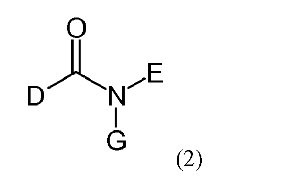 wherein:
A is selected from the group comprising linear or branched C₁-C₁₀-alkyl, linear or branched C₂-C₁₀-alkenyl and/or structural elements (A1), (A2), (A3), (A4), (A5), (A6), (A7), (A8), (A9) and/or (A10) as given as follows: 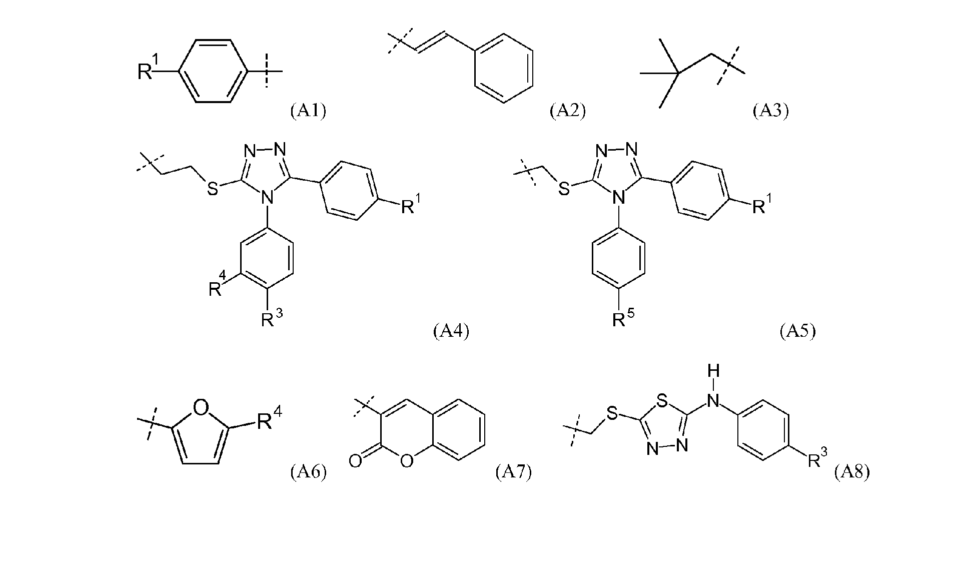 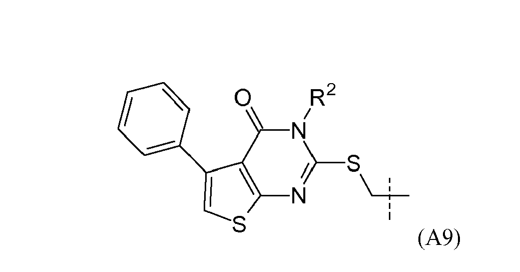 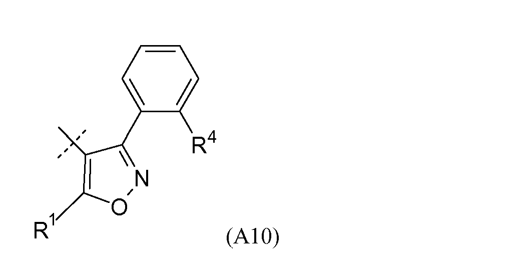
B is selected from the group comprising structural elements (B1), (B2), (B3), (B4), (B5), (B6), (B7), (B8), (B9), (B10), (B11), (B12) and/or (B13) as given as follows: 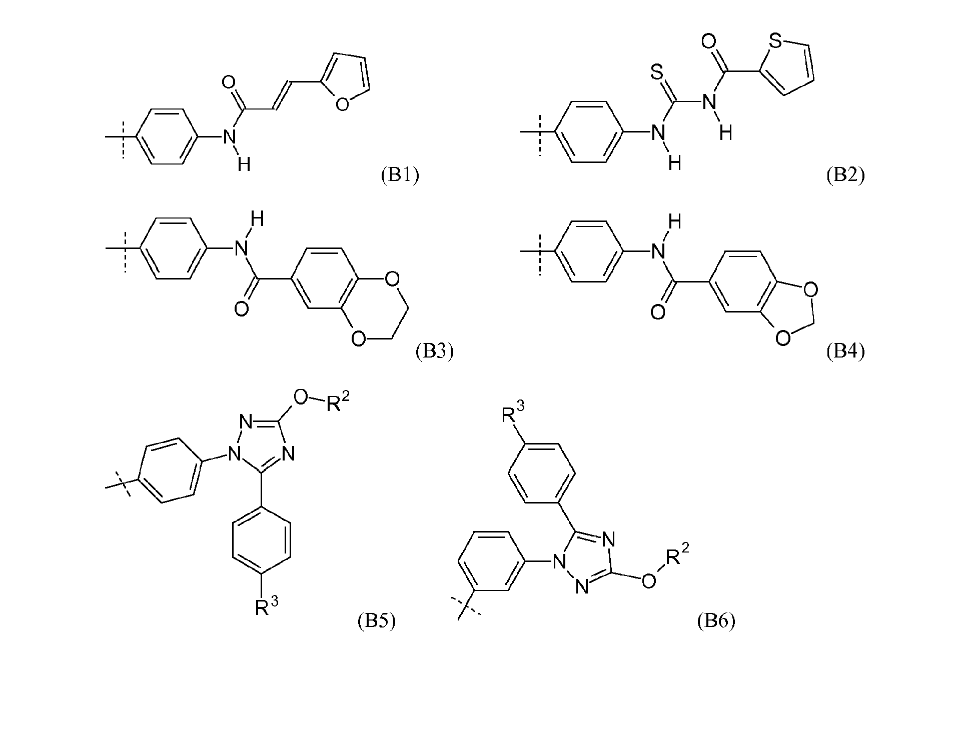 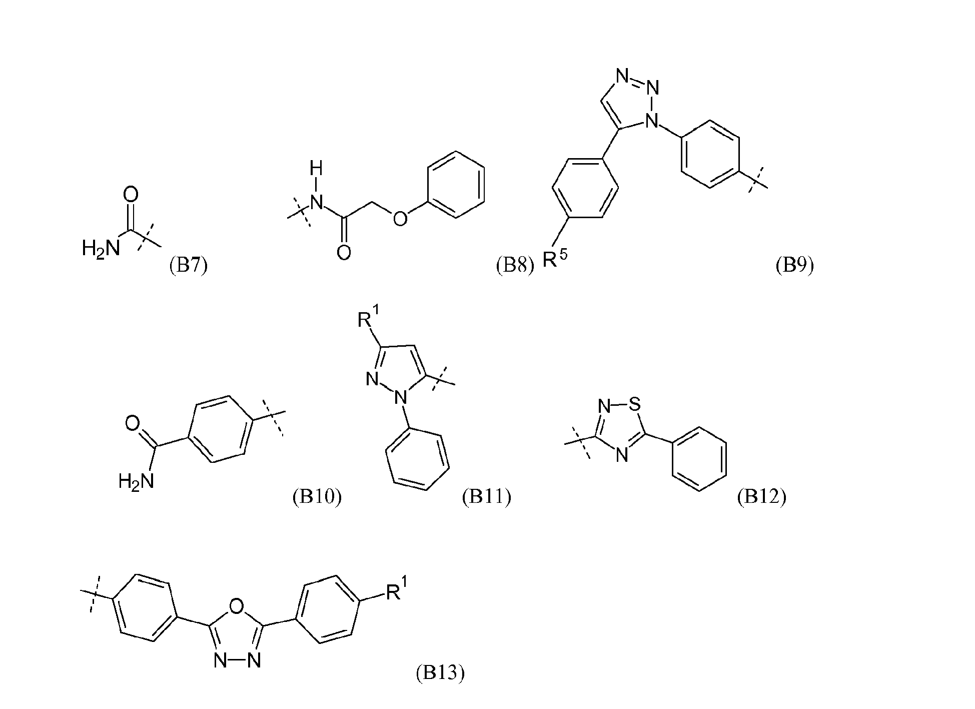
D is selected from the group comprising structural elements (D1), (D2), (D3), (D4) and/or (D5) as given as follows: 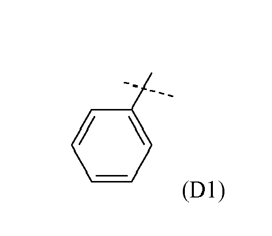 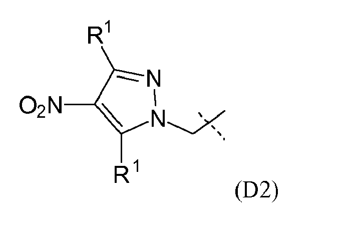 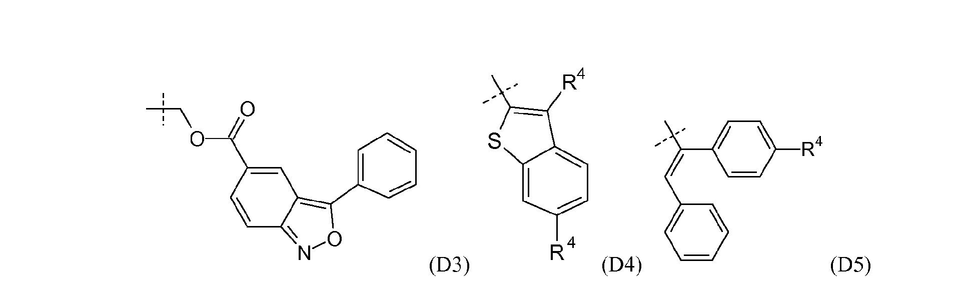
E is selected from the group comprising linear or branched C₁-C₁₀-alkyl, linear or branched C₂-C₁₀-alkenyl and/or structural elements (E1) or (E2) as given as follows: 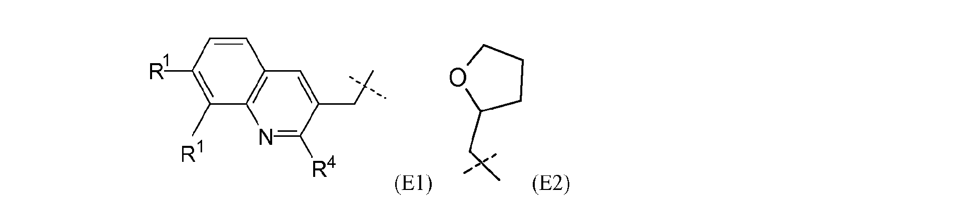
G is selected from the group comprising linear or branched C₁-C₁₀-alkyl, linear or branched C₂-C₁₀-alkenyl and/or structural elements (G1), (G2) or (G3) as given as follows: 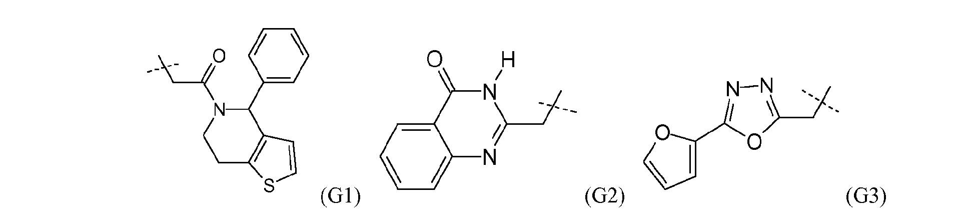 or
E and G together with the nitrogen atom to which they are attached form a 5- to 7- membered aromatic or non aromatic heterocycle selected from the group comprising structural elements (EG1) or (EG2) as given as follows: 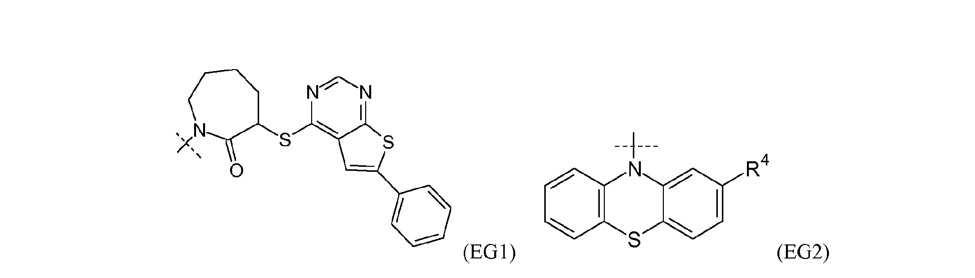 or
D and G together with the nitrogen atom to which they are attached form a 5- to 7- membered aromatic or non aromatic heterocycle (DG1) as given as follows: 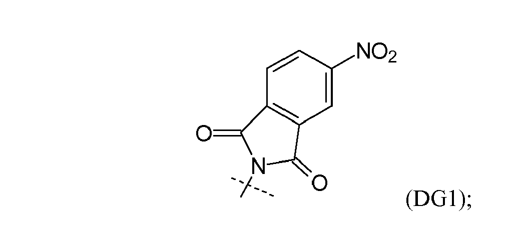
R¹ is selected, the same or each independently of the others, from the group comprising hydrogen, linear or branched C₁-C₁₀-alkyl and/or linear or branched C₂-C₁₀-alkenyl;
R² is selected, the same or each independently of the others, from the group comprising linear or branched C₁-C₁₀-alkyl and/or linear or branched C₂-C₁₀-alkenyl;
R³ is selected, the same or each independently of the others, from the group comprising linear or branched C₁-C₁₀-alkyl and/or linear or branched C₁-C₁₀-alkoxy;
R⁴ is selected, the same or each independently of the others, from the group comprising hydrogen and/or halogen, preferably selected from the group comprising Cl, Br, F;
R⁵ is selected, the same or each independently of the others, from the group comprising hydrogen and/or linear or branched C₁-C₁₀-alkoxy.

2. The pharmaceutical preparation according to claim 1, **characterized in that** the primary amide is a primary amide according to the general formula (1), wherein the structural element A is (A1) and the structural element B is selected from the group comprising (B1), (B2), (B3) and/or (B4).

3. The pharmaceutical preparation according to claim 1 or 2, **characterized in that** the primary amide is selected from the group comprising the general formulas (3) and/or (4) as indicated below and/or racemates, enantiomers, diastereomers, solvates, hydrates, and pharmaceutically acceptable salts and/or esters thereof: 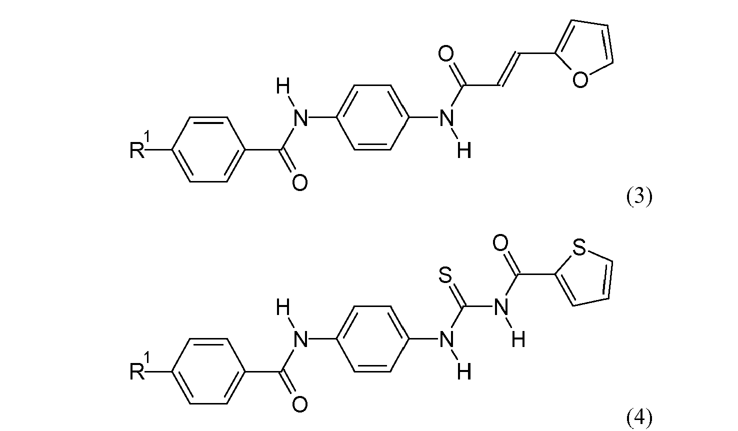 wherein:
R¹ is selected, the same or each independently of the others, from the group comprising hydrogen, linear or branched C₁-C₁₀-alkyl and/or linear or branched C₂-C₁₀-alkenyl.

4. The pharmaceutical preparation according to any of the preceding claims, **characterized in that** the primary amide is selected from the group comprising the general formulas (5) and/or (6) as indicated below and/or racemates, enantiomers, diastereomers, solvates, hydrates, and pharmaceutically acceptable salts and/or esters thereof:

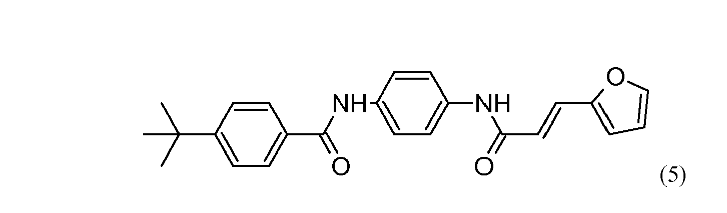

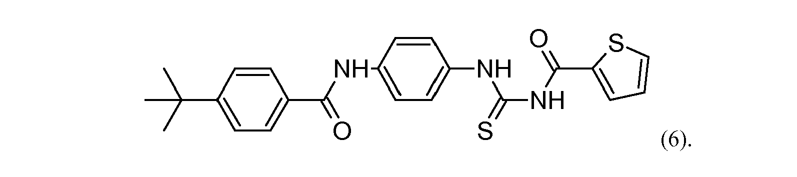

5. The pharmaceutical preparation according to any of the preceding claims, **characterized in that** the primary amide is a primary amide according to the general formula (1), wherein the structural element A is selected from the group comprising branched C₁-C₁₀-alkyl groups, (A2) and/or (A3), preferably branched C₁-C₁₀-alkyl groups or (A3), and the structural element B is selected from the group comprising (B5) and/or (B6).

6. The pharmaceutical preparation according to any of the preceding claims, **characterized in that** the primary amide is a primary amide according to the general formula (1), wherein the structural element A is (A4), and the structural element B is (B7).

7. The pharmaceutical preparation according to any of the preceding claims, **characterized in that** the primary amide is a primary amide according to the general formula (1), wherein the structural element A is (A5), and the structural element B is selected from the group comprising (B8) and/or (B10).

8. The pharmaceutical preparation according to any of the preceding claims, **characterized in that** the primary amide is a primary amide according to the general formula (1), wherein the structural element A is selected from the group comprising (A6), (A7) and/or (A10), and the structural element B is (B9).

9. The pharmaceutical preparation according to any of the preceding claims, **characterized in that** the primary and/or secondary amides are selected from the group comprising compounds (7) to (30) as indicated below and/or racemates, enantiomers, diastereomers, solvates, hydrates, and pharmaceutically acceptable salts and/or esters thereof:

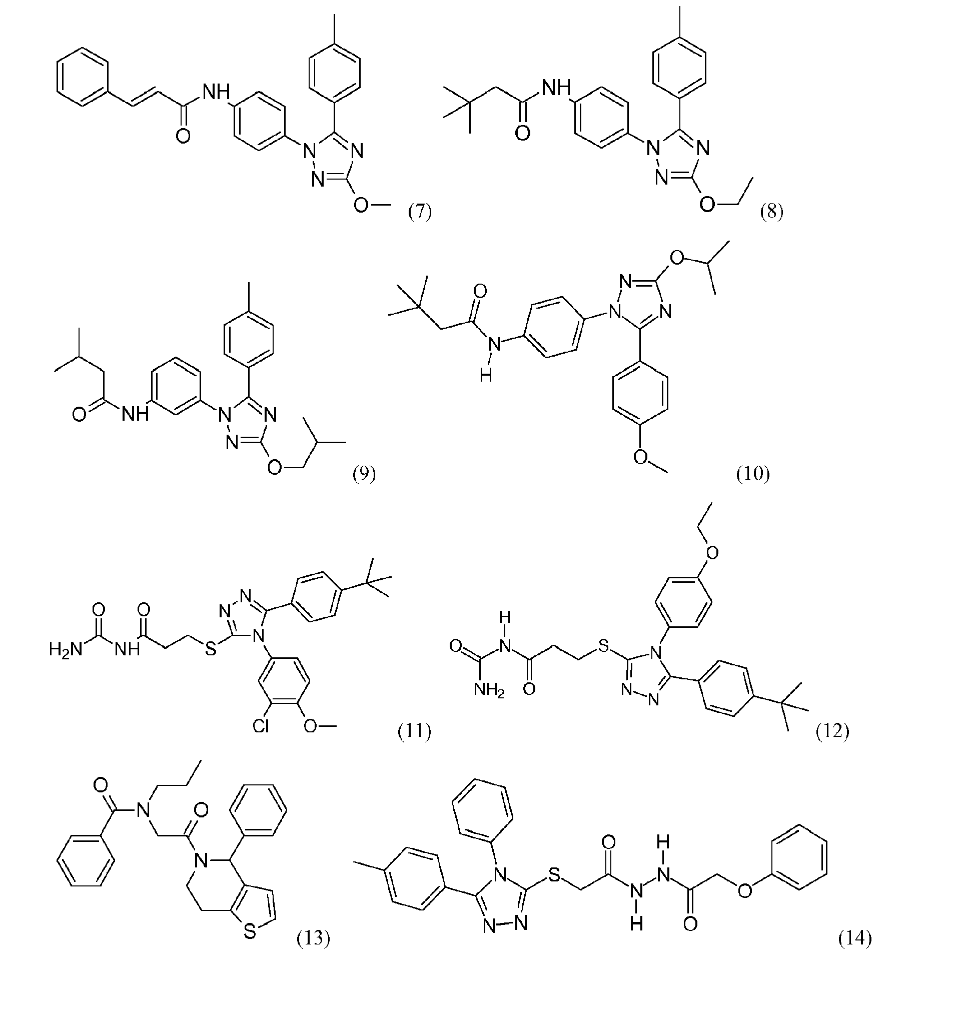

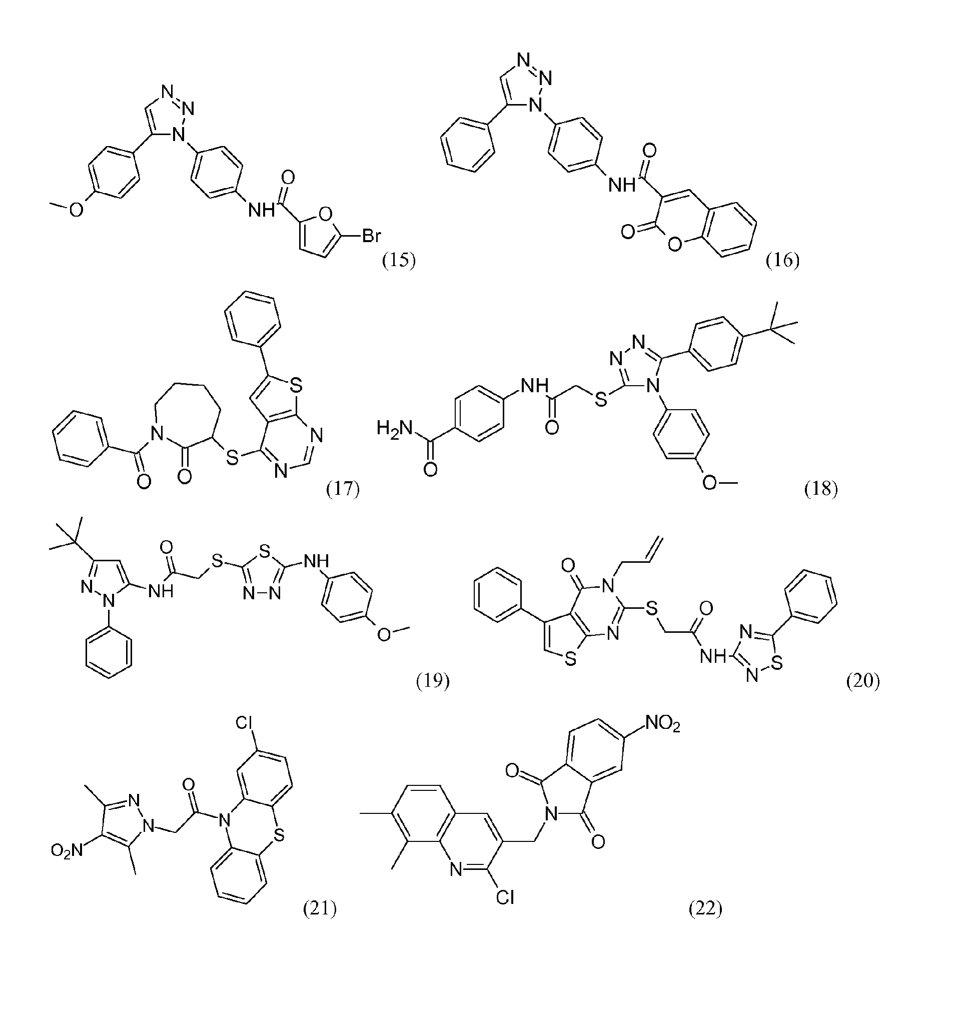

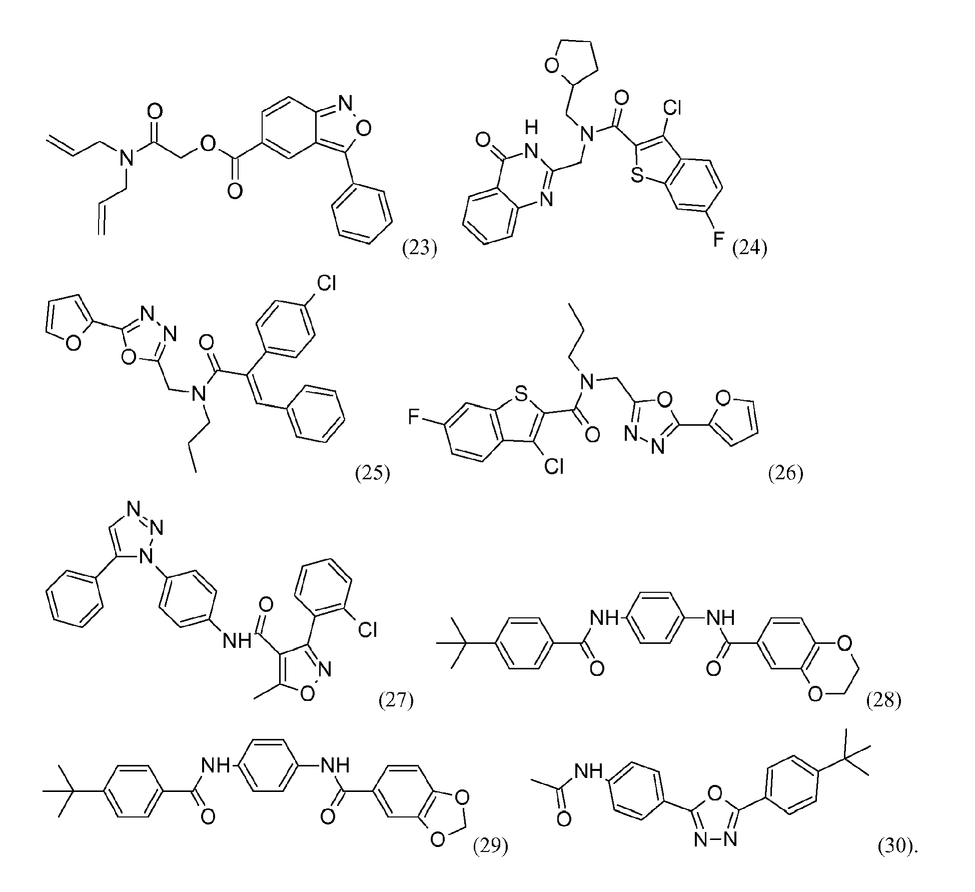

10. Primary amides according to the general formula (1) and/or a secondary amides according to the general formula (2) as indicated below and/or racemates, enantiomers, diastereomers, solvates, hydrates, and pharmaceutically acceptable salts and/or esters thereof: 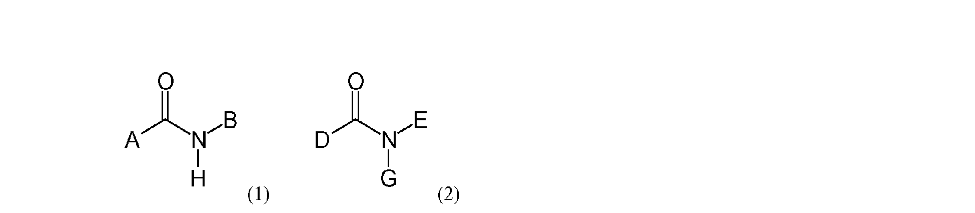 wherein:
A is selected from the group comprising structural elements linear or branched C₁-C₁₀-alkyl, linear or branched C₂-C₁₀-alkenyl and/or (A1), (A2), (A3), (A4), (A5), (A6), (A7), (A8), (A9) and/or (A10) as given as follows: 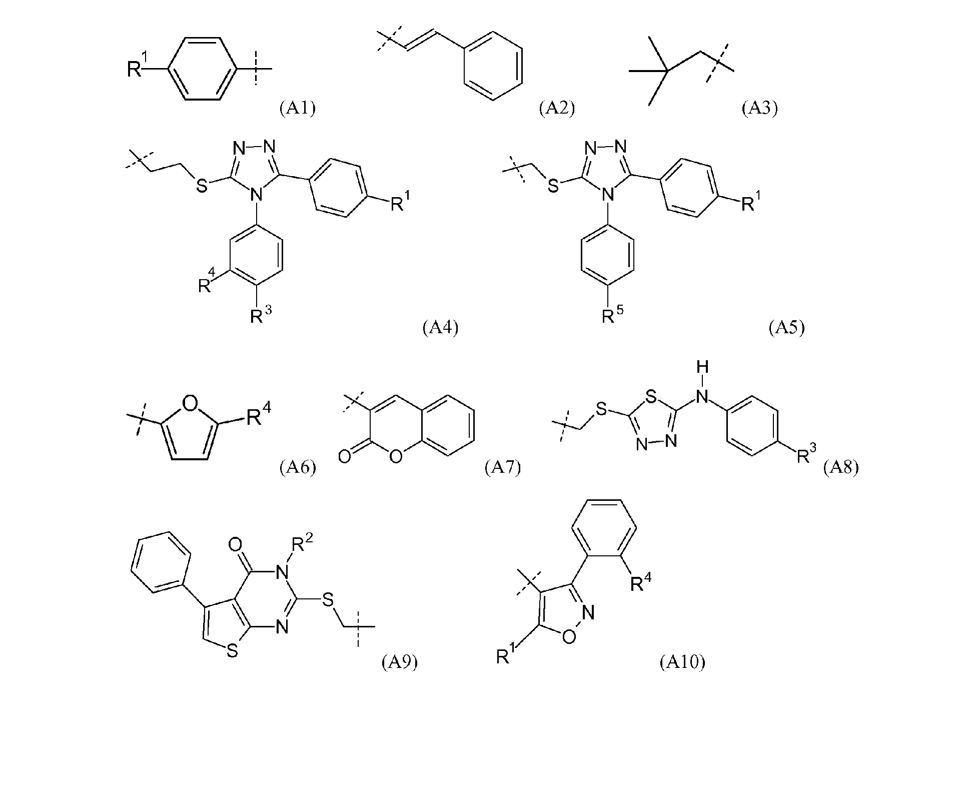
B is selected from the group comprising structural elements (B1), (B2), (B3), (B4), (B5), (B6), (B7), (B8), (B9), (B10), (B11), (B12) and/or (B13) as given as follows: 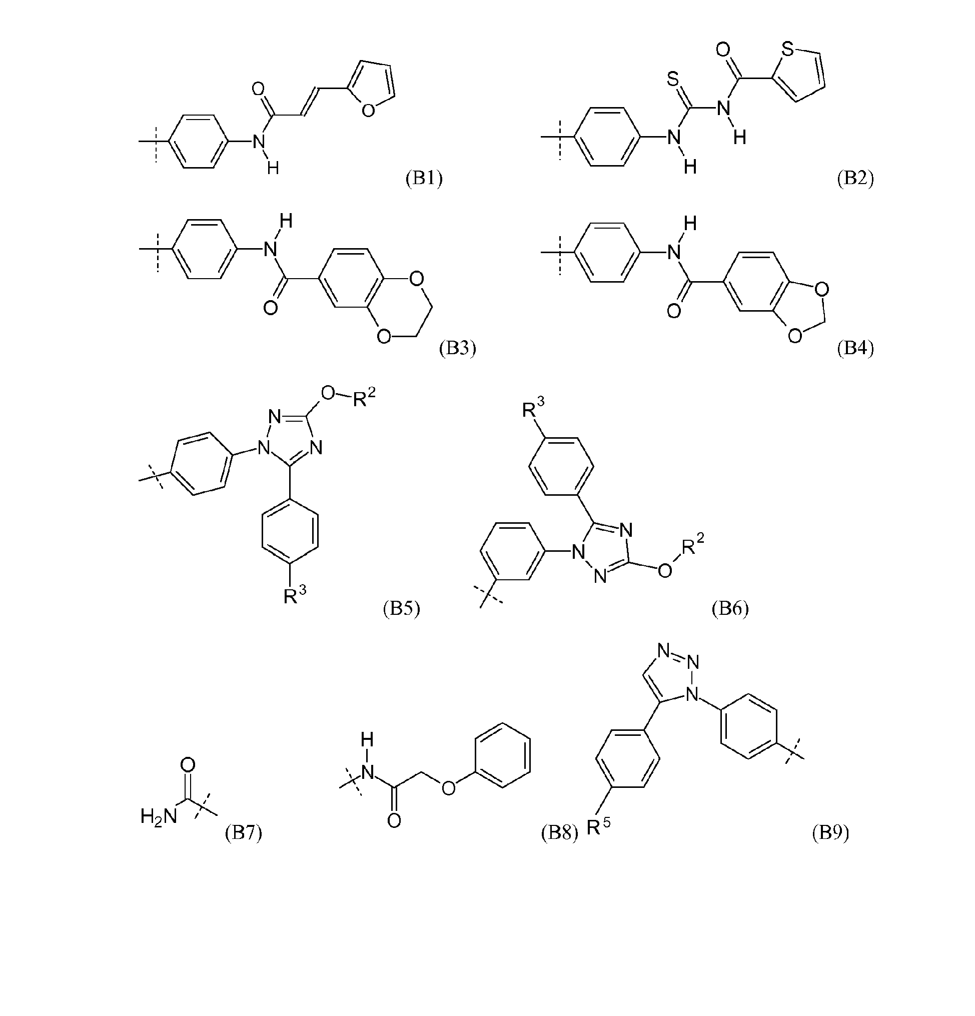 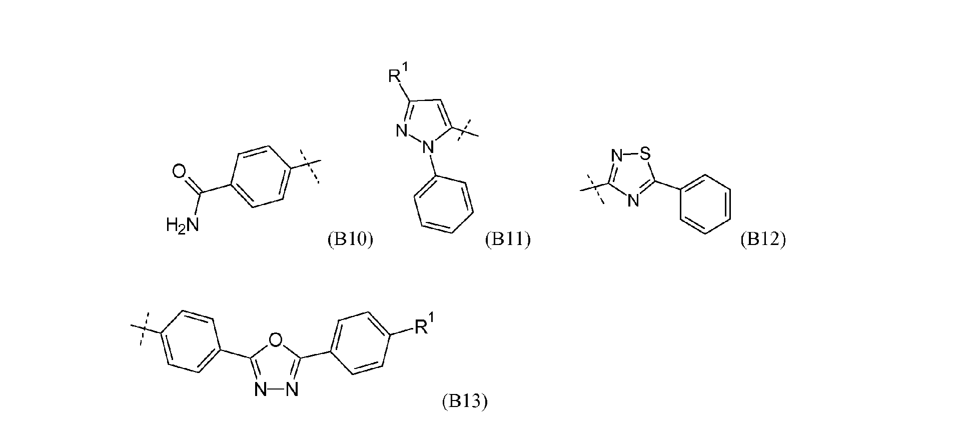
D is selected from the group comprising structural elements (D1), (D2), (D3), (D4) and/or (D5) as given as follows: 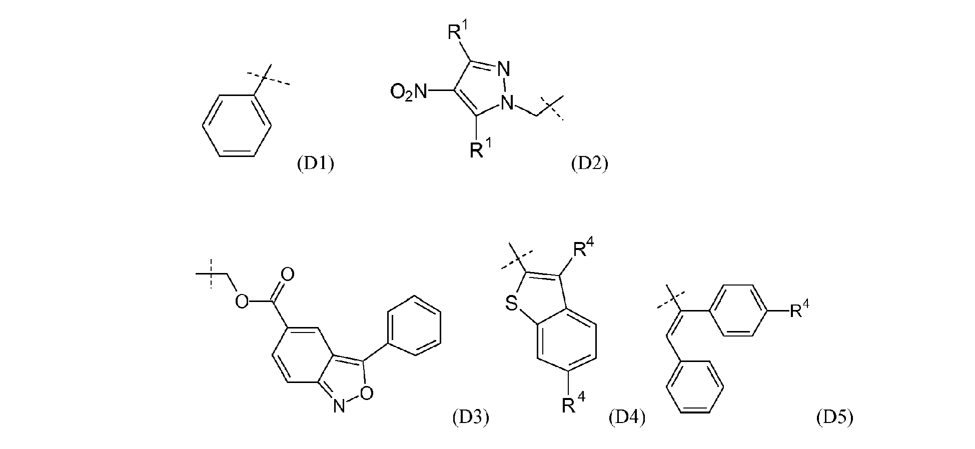
E is selected from the group comprising linear or branched C₁-C₁₀-alkyl, linear or branched C₂-C₁₀-alkenyl and/or structural elements (E1) or (E2) as given as follows: 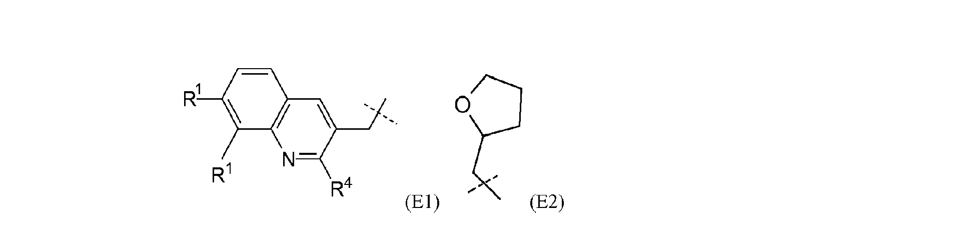
G is selected from the group comprising linear or branched C₁-C₁₀-alkyl, linear or branched C₂-C₁₀-alkenyl and/or structural elements (G1), (G2) or (G3) as given as follows: 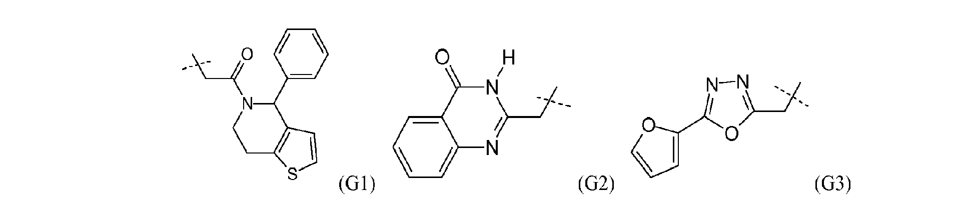 or
E and G together with the nitrogen atom to which they are attached form a 5- to 7- membered aromatic or non aromatic heterocycle selected from the group comprising structural elements (EG1) or (EG2) as given as follows: 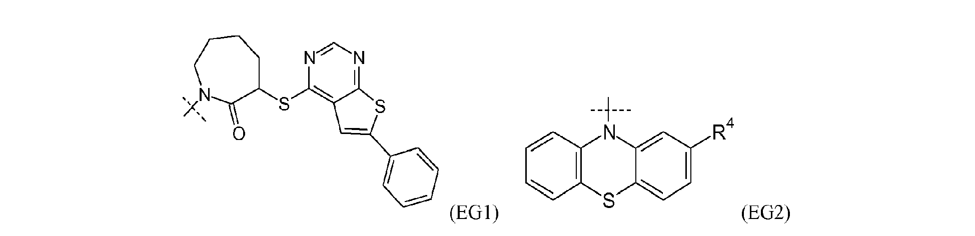 or
D and G together with the nitrogen atom to which they are attached form a 5- to 7- membered aromatic or non aromatic heterocycle (DG1) as given as follows: 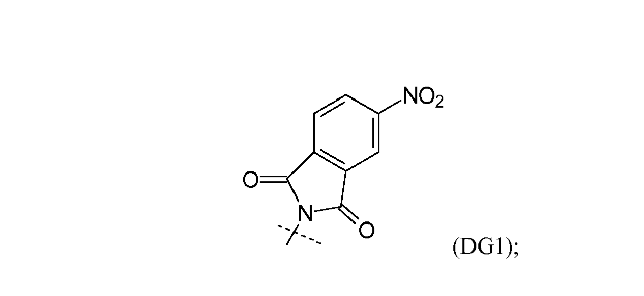
R¹ is selected, the same or each independently of the others, from the group comprising hydrogen, linear or branched C₁-C₁₀-alkyl and/or linear or branched C₂-C₁₀-alkenyl;
R² is selected, the same or each independently of the others, from the group comprising linear or branched C₁-C₁₀-alkyl and/or linear or branched C₂-C₁₀-alkenyl;
R³ is selected, the same or each independently of the others, from the group comprising linear or branched C₁-C₁₀-alkyl and/or linear or branched C₁-C₁₀-alkoxy;
R⁴ is selected, the same or each independently of the others, from the group comprising hydrogen and/or halogen, preferably selected from the group comprising Cl, Br, F;
R⁵ is selected, the same or each independently of the others, from the group comprising hydrogen and/or linear or branched C₁-C₁₀-alkoxy,
for use as a medicament.

11. Primary amides according to the general formula (1) and/or a secondary amides according to the general formula (2) as indicated below and/or racemates, enantiomers, diastereomers, solvates, hydrates, and pharmaceutically acceptable salts and/or esters thereof: 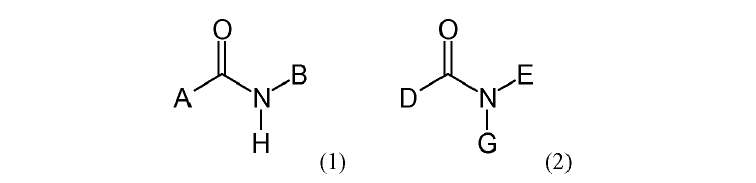 wherein:
A is selected from the group comprising linear or branched C₁-C₁₀-alkyl, linear or branched C₂-C₁₀-alkenyl and/or structural elements (A1), (A2), (A3), (A4), (A5), (A6), (A7), (A8), (A9) and/or (A10) as given as follows: 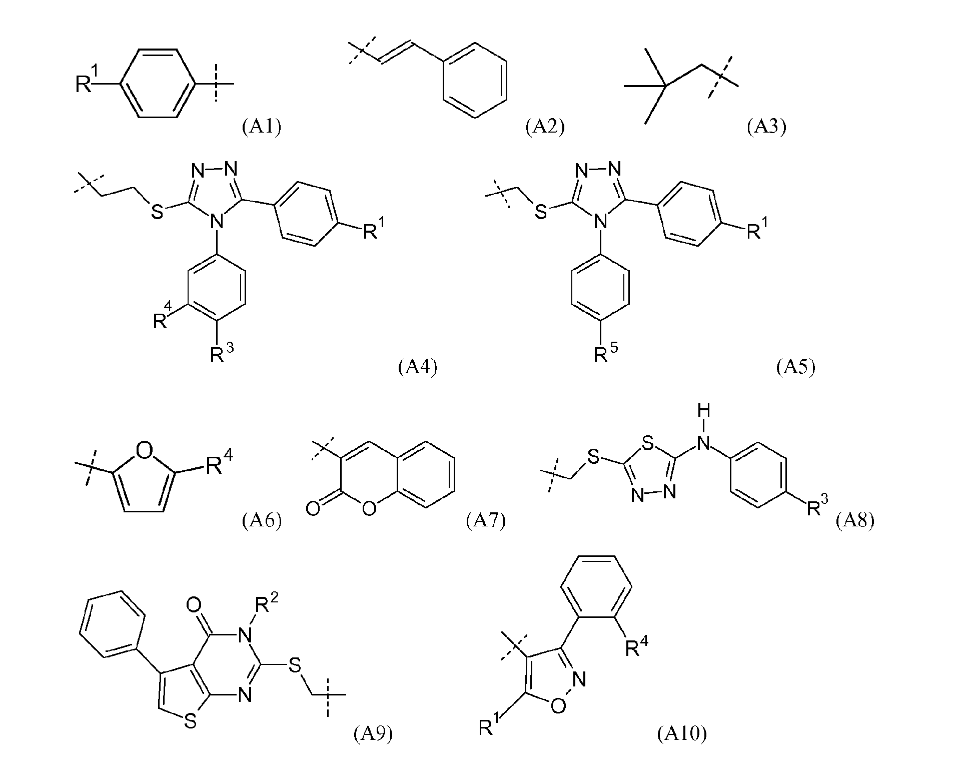
B is selected from the group comprising structural elements (B1), (B2), (B3), (B4), (B5), (B6), (B7), (B8), (B9), (B10), (B11), (B12) and/or (B13) as given as follows: 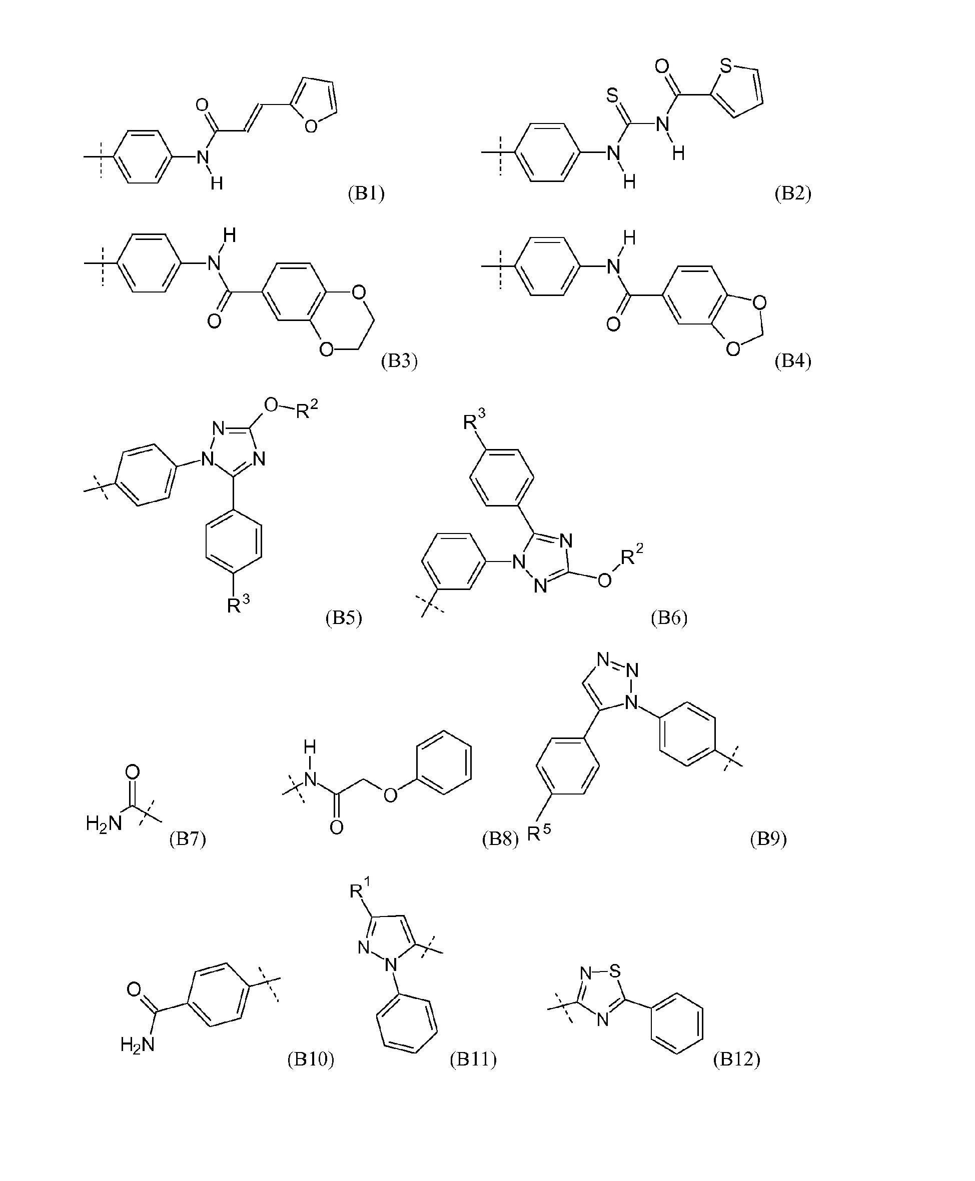 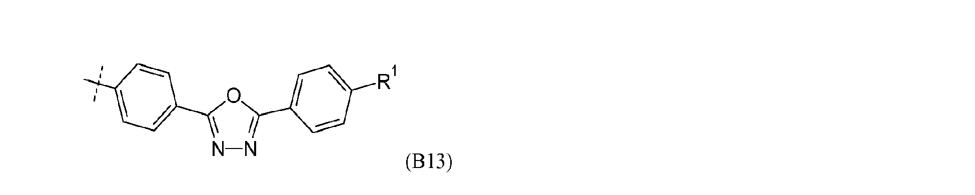
D is selected from the group comprising structural elements (D1), (D2), (D3), (D4) and/or (D5) as given as follows: 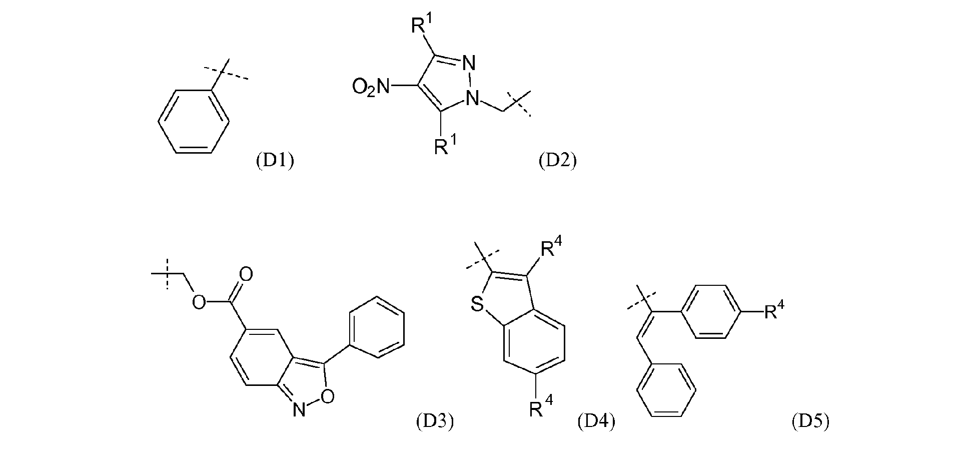
E is selected from the group comprising linear or branched C₁-C₁₀-alkyl, linear or branched C₂-C₁₀-alkenyl and/or structural elements (E1) or (E2) as given as follows: 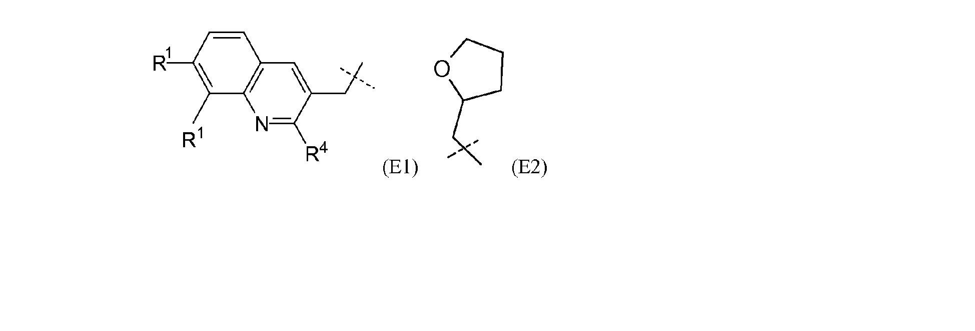
G is selected from the group comprising linear or branched C₁-C₁₀-alkyl, linear or branched C₂-C₁₀-alkenyl and/or structural elements (G1), (G2) or (G3) as given as follows: 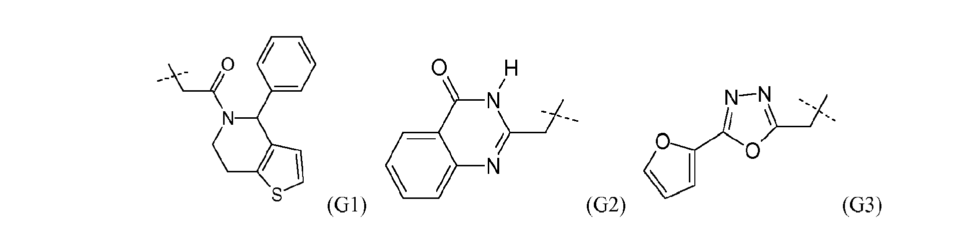 or
E and G together with the nitrogen atom to which they are attached form a 5- to 7- membered aromatic or non aromatic heterocycle selected from the group comprising structural elements (EG1) or (EG2) as given as follows: 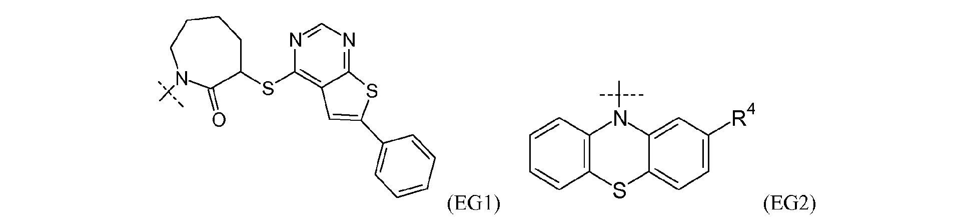 or
D and G together with the nitrogen atom to which they are attached form a 5- to 7- membered aromatic or non aromatic heterocycle (DG1) as given as follows: 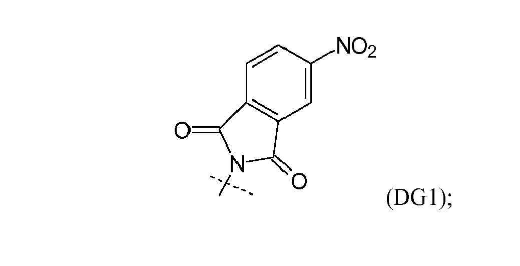
R¹ is selected, the same or each independently of the others, from the group comprising hydrogen, linear or branched C₁-C₁₀-alkyl and/or linear or branched C₂-C₁₀-alkenyl;
R² is selected, the same or each independently of the others, from the group comprising linear or branched C₁-C₁₀-alkyl and/or linear or branched C₂-C₁₀-alkenyl;
R³ is selected, the same or each independently of the others, from the group comprising linear or branched C₁-C₁₀-alkyl and/or linear or branched C₁-C₁₀-alkoxy;
R⁴ is selected, the same or each independently of the others, from the group comprising hydrogen and/or halogen, preferably selected from the group comprising Cl, Br, F;
R⁵ is selected, the same or each independently of the others, from the group comprising hydrogen and/or linear or branched C₁-C₁₀-alkoxy,
for use in the therapeutic and/or prophylactic treatment of a disease selected from the group comprising autoimmune diseases such as rheumatoid arthritis, multiple sclerosis, psoriasis, Crohn's disease, allergies, mastocytosis, tumor diseases such as lung or bronchial cancer, colon, gastrointestinal, pancreatic or rectal cancer, prostate cancer, lymphatic cancer or leukemia, bladder or kidney cancer, breast and/or ovarian cancer, diseases and pathological conditions that are linked to a hyperfunction of cytohesin dependent receptor tyrosine kinases such as the ErbB receptors and other growth factor receptors selected from the group comprising developmental syndromes such as craniosynostosis, dwarfism, Hirschsprung disease, multiple endocrine neoplasia, vascular dysmorphogenesis and/or disorders of the lymphatic system, diseases and pathological conditions that are linked to a regulation of the insulin and/or insulin-like growth factor (IGF) signalling pathway selected from the group comprising obesity, cell aging, age-related cell damage, especially in the liver and/or the pancreas, age-related pathological conditions of liver and/or pancreatic cells, age-related functional disorders in the liver and/or pancreas, cell stress, especially oxidative stress, especially stress induced as a result of increased sugar metabolization, and/or apoptosis, especially β-cell apoptosis, for immunosuppression, for example, in cases of organ transplants, and/or for inhibition of angiogenesis.

12. Use of primary amides according to the general formula (1) and/or a secondary amides according to the general formula (2) as indicated below and/or racemates, enantiomers, diastereomers, solvates, hydrates, and pharmaceutically acceptable salts and/or esters thereof: 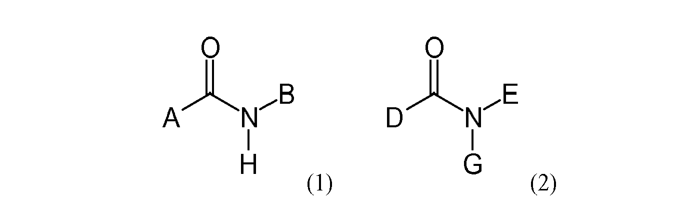 wherein:
A is selected from the group comprising linear or branched C₁-C₁₀-alkyl, linear or branched C₂-C₁₀-alkenyl and/or structural elements (A1), (A2), (A3), (A4), (A5), (A6), (A7), (A8), (A9) and/or (A10) as given as follows: 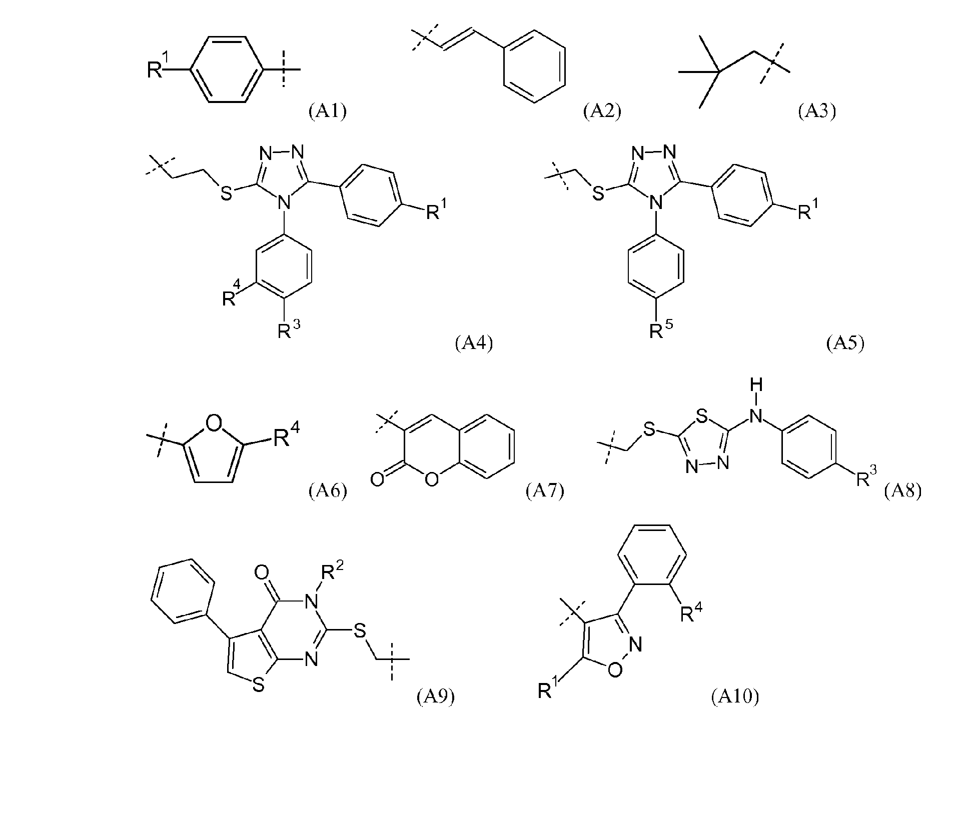
B is selected from the group comprising structural elements (B1), (B2), (B3), (B4), (B5), (B6), (B7), (B8), (B9), (B10), (B11), (B12) and/or (B13) as given as follows: 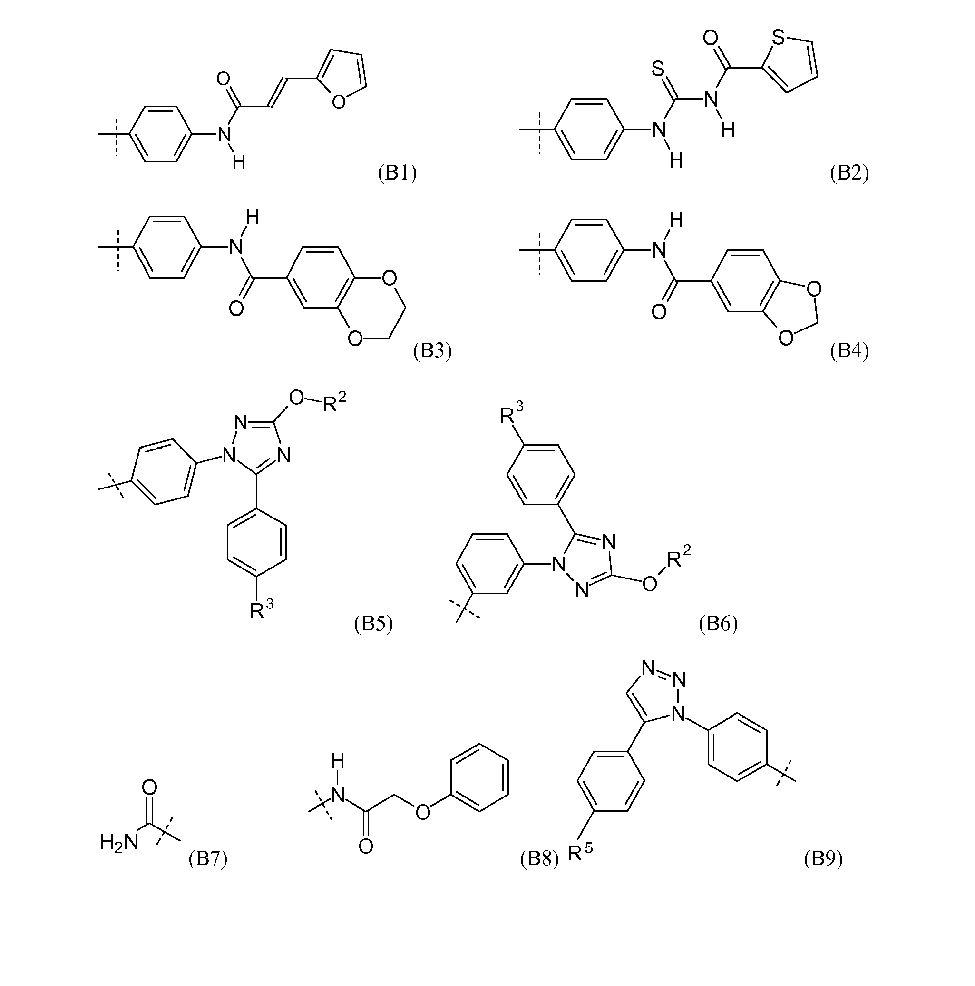 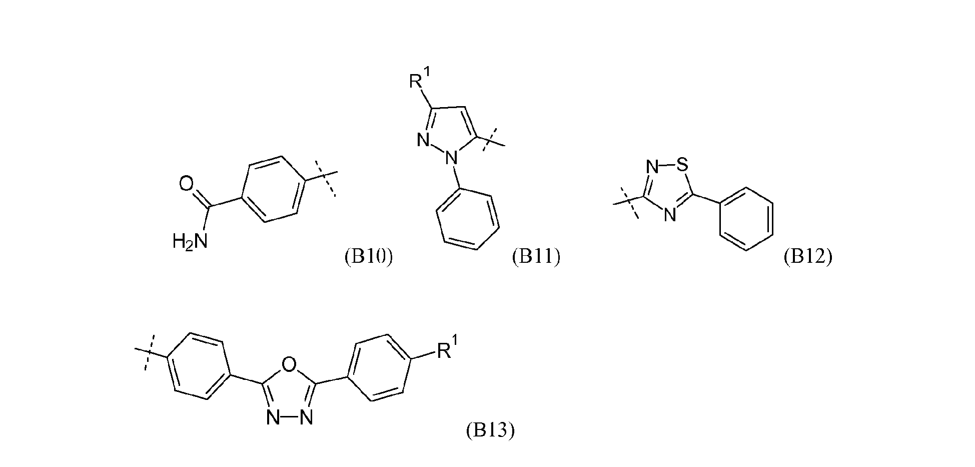
D is selected from the group comprising structural elements (D1), (D2), (D3), (D4) and/or (D5) as given as follows: 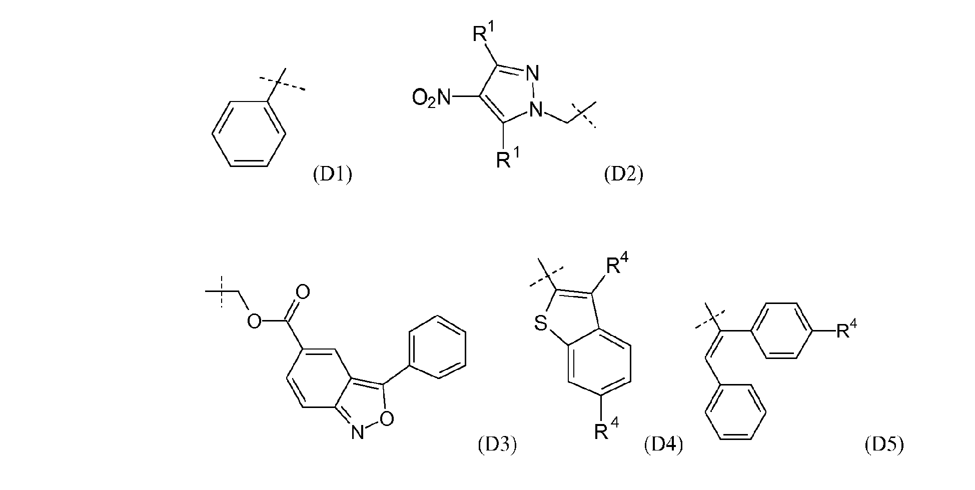
E is selected from the group comprising linear or branched C₁-C₁₀-alkyl, linear or branched C₂-C₁₀-alkenyl and/or structural elements (E1) or (E2) as given as follows: 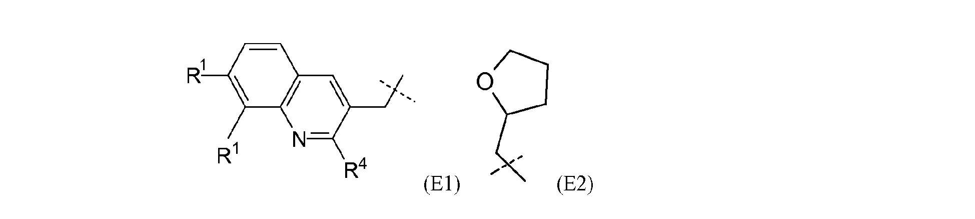
G is selected from the group comprising linear or branched C₁-C₁₀-alkyl, linear or branched C₂-C₁₀-alkenyl and/or structural elements (G1), (G2) or (G3) as given as follows: 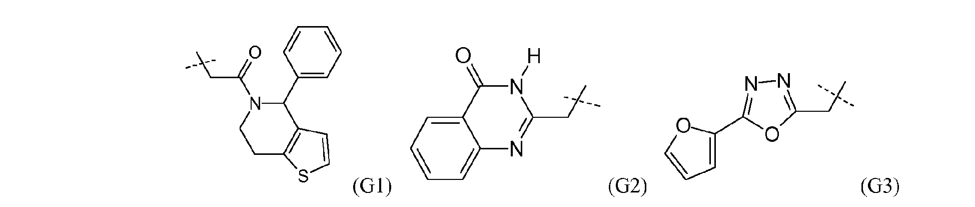 or
E and G together with the nitrogen atom to which they are attached form a 5- to 7- membered aromatic or non aromatic heterocycle selected from the group comprising structural elements (EG1) or (EG2) as given as follows: 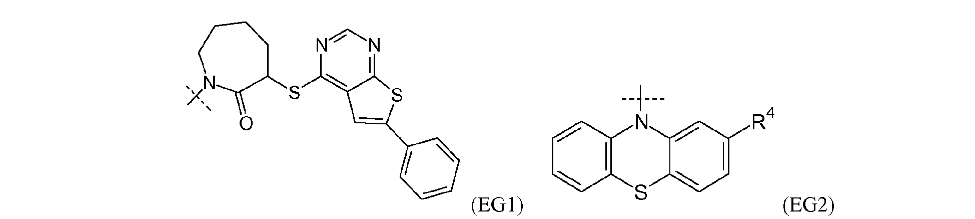 or
D and G together with the nitrogen atom to which they are attached form a 5- to 7- membered aromatic or non aromatic heterocycle (DG1) as given as follows: 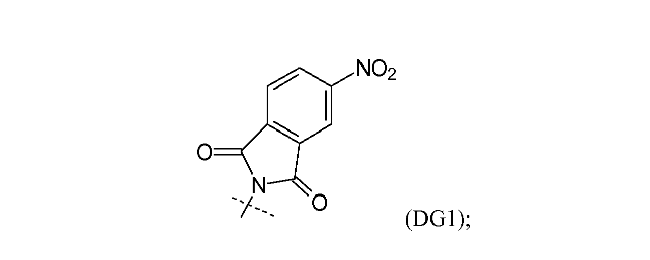
R¹ is selected, the same or each independently of the others, from the group comprising hydrogen, linear or branched C₁-C₁₀-alkyl and/or linear or branched C₂-C₁₀-alkenyl;
R² is selected, the same or each independently of the others, from the group comprising linear or branched C₁-C₁₀-alkyl and/or linear or branched C₂-C₁₀-alkenyl;
R³ is selected, the same or each independently of the others, from the group comprising linear or branched C₁-C₁₀-alkyl and/or linear or branched C₁-C₁₀-alkoxy;
R⁴ is selected, the same or each independently of the others, from the group comprising hydrogen and/or halogen, preferably selected from the group comprising Cl, Br, F;
R⁵ is selected, the same or each independently of the others, from the group comprising hydrogen and/or linear or branched C₁-C₁₀-alkoxy,
for the manufacture of a medicament.

13. The use of primary amides according to the general formula (1) and/or a secondary amides according to the general formula (2) as indicated below and/or racemates, enantiomers, diastereomers, solvates, hydrates, and pharmaceutically acceptable salts and/or esters thereof: 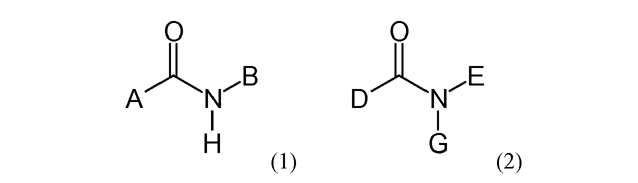 wherein:
A is selected from the group comprising linear or branched C₁-C₁₀-alkyl, linear or branched C₂-C₁₀-alkenyl and/or structural elements (A1), (A2), (A3), (A4), (A5), (A6), (A7), (A8), (A9) and/or (A10) as given as follows: 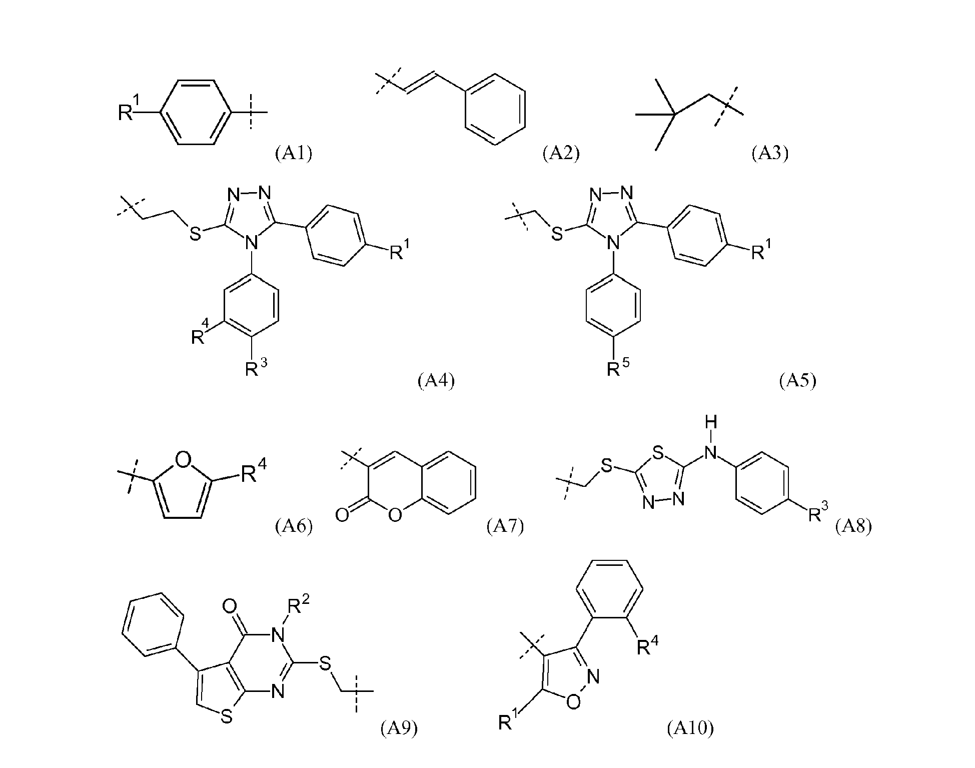
B is selected from the group comprising structural elements (B1), (B2), (B3), (B4), (B5), (B6), (B7), (B8), (B9), (B10), (B11), (B12) and/or (B13) as given as follows: 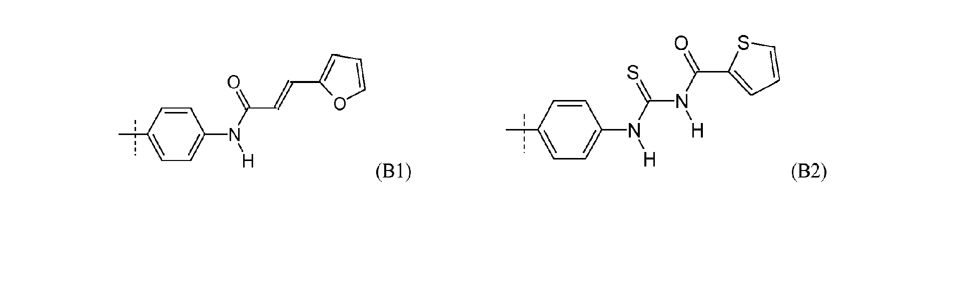 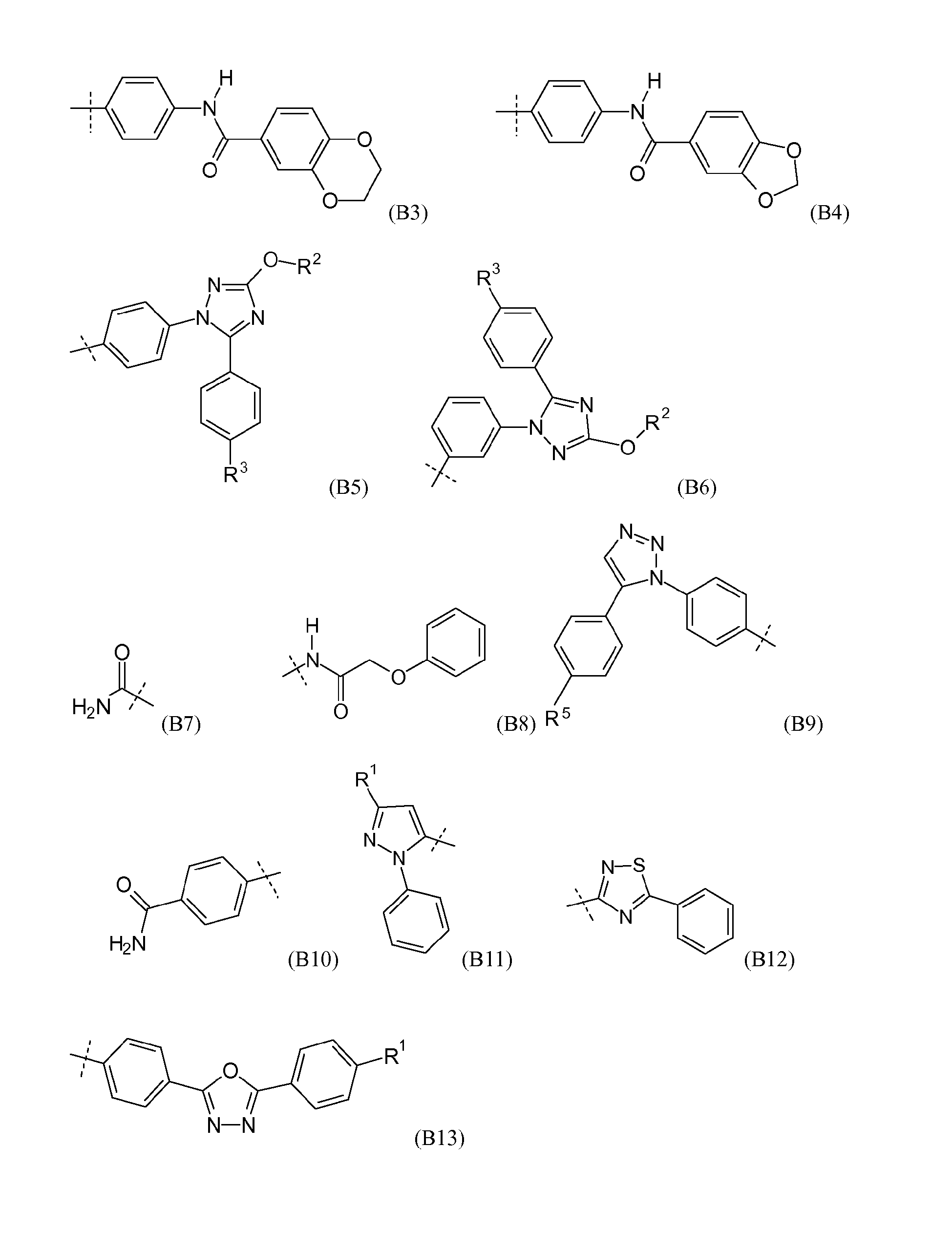
D is selected from the group comprising structural elements (D1), (D2), (D3), (D4) and/or (D5) as given as follows: 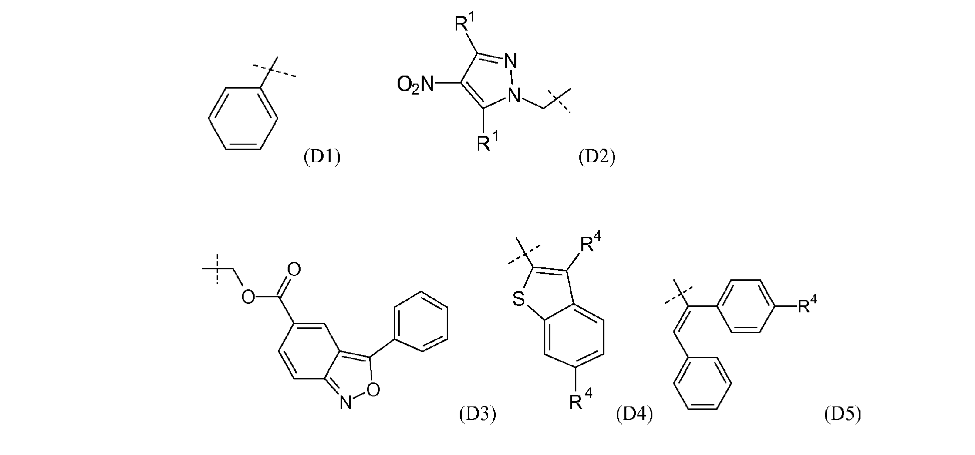
E is selected from the group comprising linear or branched C₁-C₁₀-alkyl, linear or branched C₂-C₁₀-alkenyl and/or structural elements (E1) or (E2) as given as follows: 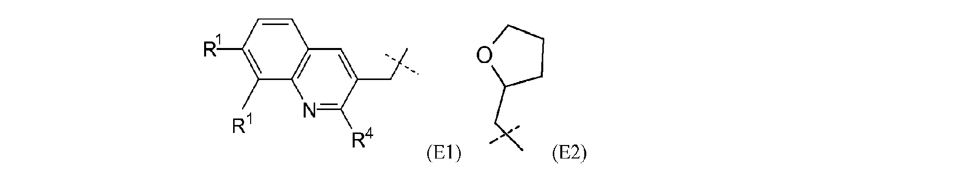
G is selected from the group comprising linear or branched C₁-C₁₀-alkyl, linear or branched C₂-C₁₀-alkenyl and/or structural elements (G1), (G2) or (G3) as given as follows: 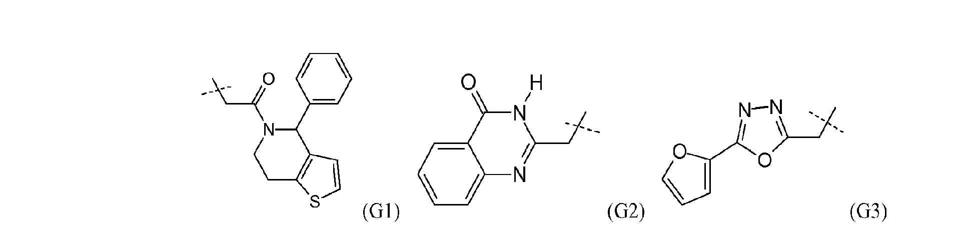 or
E and G together with the nitrogen atom to which they are attached form a 5- to 7- membered aromatic or non aromatic heterocycle selected from the group comprising structural elements (EG1) or (EG2) as given as follows: 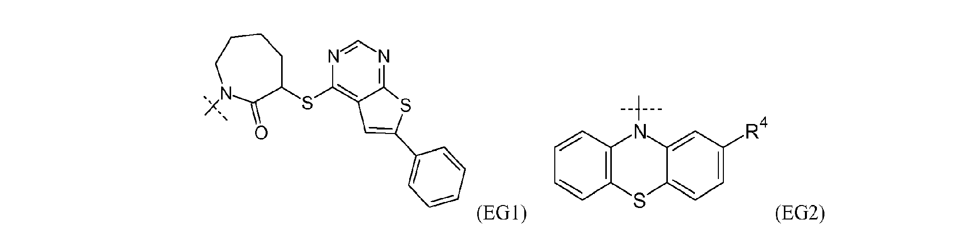 or
D and G together with the nitrogen atom to which they are attached form a 5- to 7- membered aromatic or non aromatic heterocycle (DG1) as given as follows: 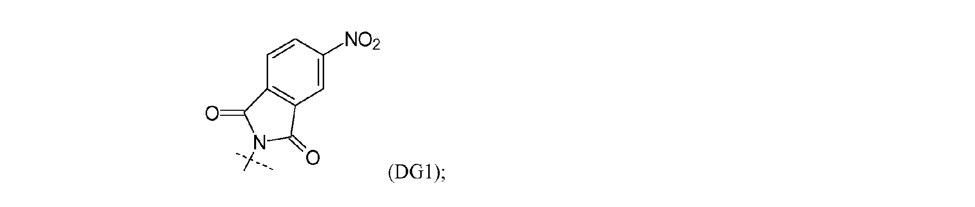
R¹ is selected, the same or each independently of the others, from the group comprising hydrogen, linear or branched C₁-C₁₀-alkyl and/or linear or branched C₂-C₁₀-alkenyl;
R² is selected, the same or each independently of the others, from the group comprising linear or branched C₁-C₁₀-alkyl and/or linear or branched C₂-C₁₀-alkenyl;
R³ is selected, the same or each independently of the others, from the group comprising linear or branched C₁-C₁₀-alkyl and/or linear or branched C₁-C₁₀-alkoxy;
R⁴ is selected, the same or each independently of the others, from the group comprising hydrogen and/or halogen, preferably selected from the group comprising Cl, Br, F;
R⁵ is selected, the same or each independently of the others, from the group comprising hydrogen and/or linear or branched C₁-C₁₀-alkoxy,
for the manufacture of a pharmaceutical preparation for the therapeutic and/or preventive treatment of a disease selected from the group comprising autoimmune diseases such as rheumatoid arthritis, multiple sclerosis, psoriasis, Crohn's disease, allergies, mastocytosis, tumor diseases such as lung or bronchial cancer, colon, gastrointestinal, pancreatic or rectal cancer, prostate cancer, lymphatic cancer or leukemia, bladder or kidney cancer, breast and/or ovarian cancer, diseases and pathological conditions that are linked to a hyperfunction of cytohesin dependent receptor tyrosine kinases such as the ErbB receptors and other growth factor receptors selected from the group comprising developmental syndromes such as craniosynostosis, dwarfism, Hirschsprung disease, multiple endocrine neoplasia, vascular dysmorphogenesis and/or disorders of the lymphatic system, diseases and pathological conditions that are linked to a regulation of the insulin and/or insulin-like growth factor (IGF) signalling pathway selected from the group comprising obesity, cell aging, age-related cell damage, especially in the liver and/or the pancreas, age-related pathological conditions of liver and/or pancreatic cells, age-related functional disorders in the liver and/or pancreas, cell stress, especially oxidative stress, especially stress induced as a result of increased sugar metabolization, and/or apoptosis, especially β-cell apoptosis, for immunosuppression, for example, in cases of organ transplants, and/or for inhibition of angiogenesis.

14. The use of primary amides according to the general formula (1) and/or a secondary amides according to the general formula (2) as indicated below and/or racemates, enantiomers, diastereomers, solvates, hydrates, and pharmaceutically acceptable salts and/or esters thereof: 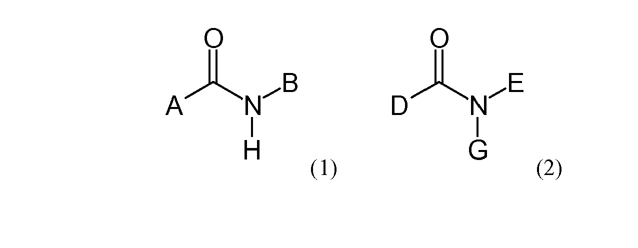 wherein:
A is selected from the group comprising linear or branched C₁-C₁₀-alkyl, linear or branched C₂-C₁₀-alkenyl and/or structural elements (A1), (A2), (A3), (A4), (A5), (A6), (A7), (A8), (A9) and/or (A10) as given as follows: 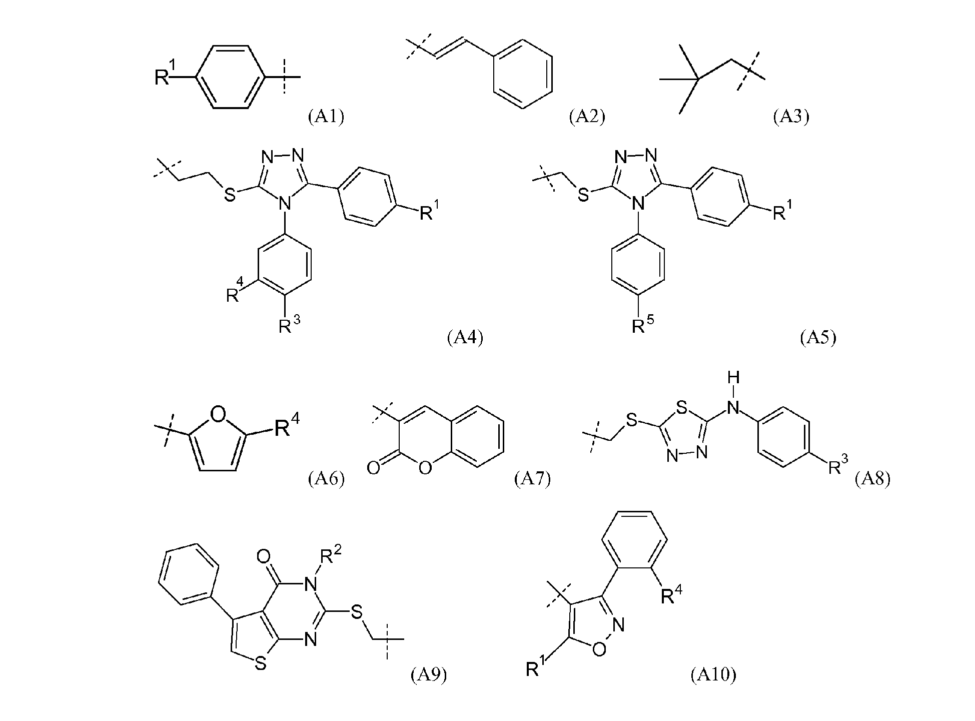
B is selected from the group comprising structural elements (B1), (B2), (B3), (B4), (B5), (B6), (B7), (B8), (B9), (B10), (B11), (B12) and/or (B13) as given as follows: 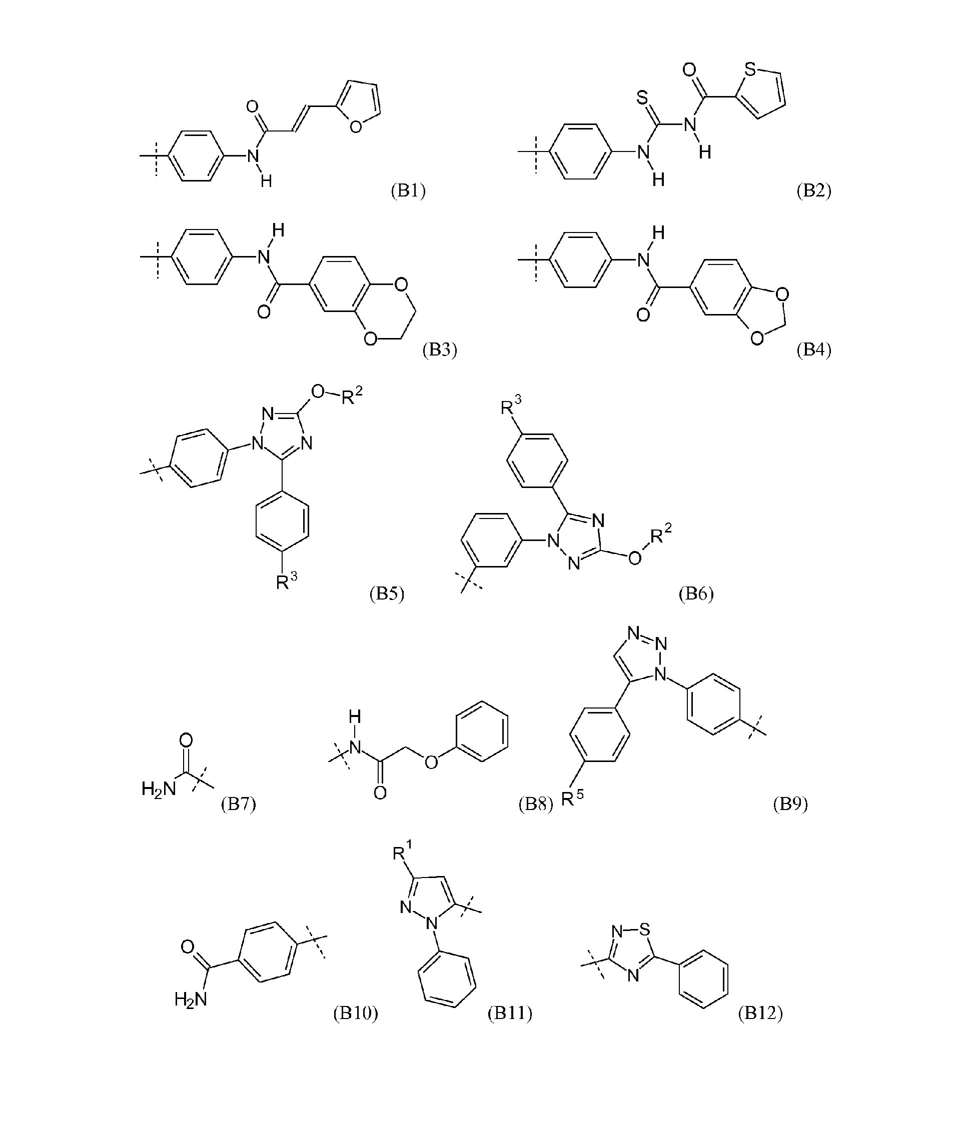 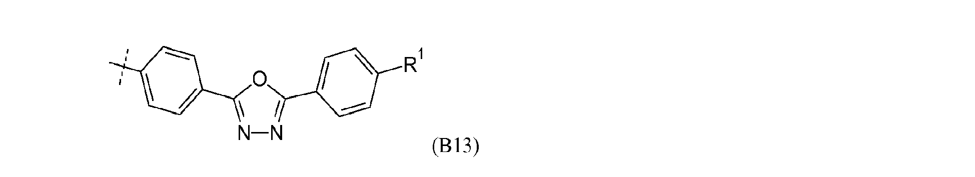
D is selected from the group comprising structural elements (D1), (D2), (D3), (D4) and/or (D5) as given as follows: 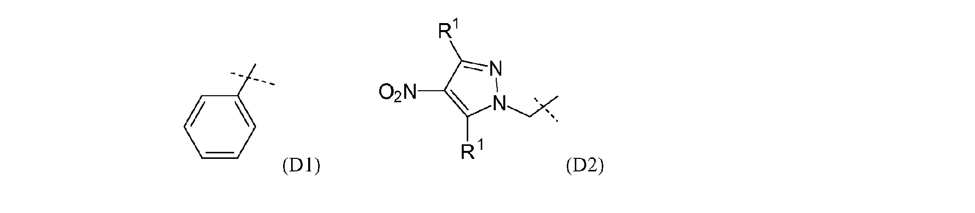 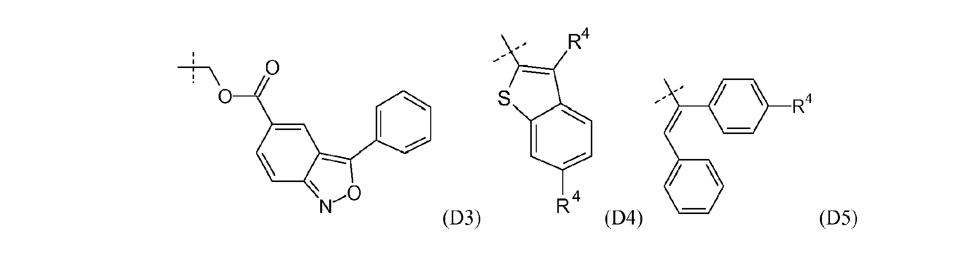
E is selected from the group comprising linear or branched C₁-C₁₀-alkyl, linear or branched C₂-C₁₀-alkenyl and/or structural elements (E1) or (E2) as given as follows: 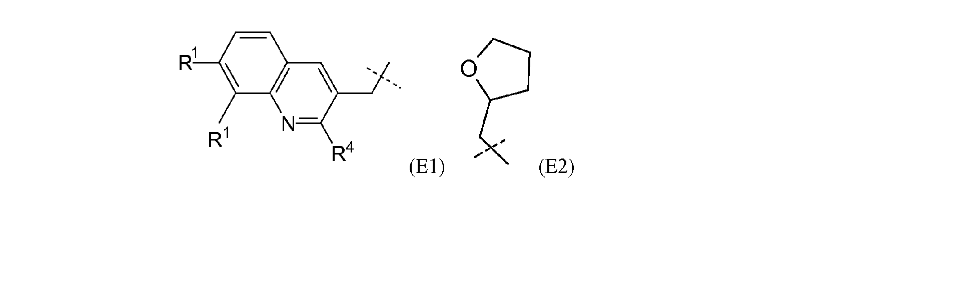
G is selected from the group comprising linear or branched C₁-C₁₀-alkyl, linear or branched C₂-C₁₀-alkenyl and/or structural elements (G1), (G2) or (G3) as given as follows: 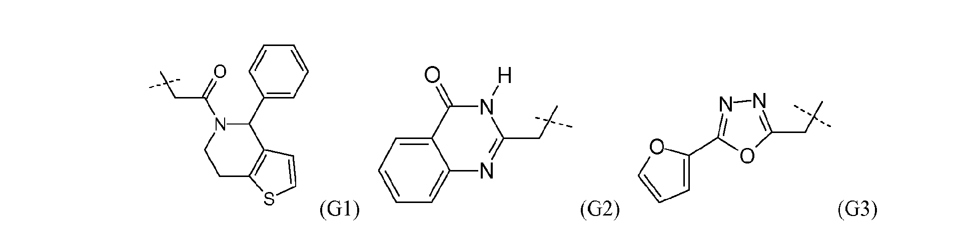 or
E and G together with the nitrogen atom to which they are attached form a 5- to 7- membered aromatic or non aromatic heterocycle selected from the group comprising structural elements (EG1) or (EG2) as given as follows: 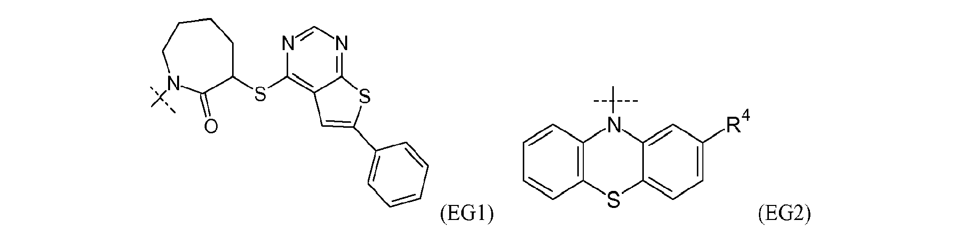 or
D and G together with the nitrogen atom to which they are attached form a 5- to 7- membered aromatic or non aromatic heterocycle (DG1) as given as follows: 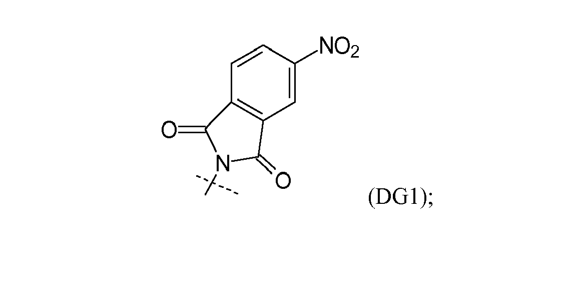
R¹ is selected, the same or each independently of the others, from the group comprising hydrogen, linear or branched C₁-C₁₀-alkyl and/or linear or branched C₂-C₁₀-alkenyl;
R² is selected, the same or each independently of the others, from the group comprising linear or branched C₁-C₁₀-alkyl and/or linear or branched C₂-C₁₀-alkenyl;
R³ is selected, the same or each independently of the others, from the group comprising linear or branched C₁-C₁₀-alkyl and/or linear or branched C₁-C₁₀-alkoxy;
R⁴ is selected, the same or each independently of the others, from the group comprising hydrogen and/or halogen, preferably selected from the group comprising Cl, Br, F;
R⁵ is selected, the same or each independently of the others, from the group comprising hydrogen and/or linear or branched C₁-C₁₀-alkoxy,
for chemically inducing longevity or increasing the lifespan of mammals.

15. Use of primary amides and/or secondary amides selected from the group comprising the general formulas (1) and/or (2) according to any of the preceding claims **characterized in that** the pharmaceutical preparation is formulated for oral or intravenous administration.
